# EUROPEAN PATENT SPECIFICATION

(11) **EP 0 555 361 B1**
(45) Date of publication and mention of the grant of the patent: **11.01.1995**
(21) Application number: 91920645.8
(22) Date of filing: 28.10.1991
(51) Int. Cl.: C09B 69/10, C08J 3/09

(54) **POWDER COLORANT COMPOSITIONS AND PROCESS FOR THE PREPARATION THEREOF**
PULVERFÖRMIGE FARBPRÄPARATE SOWIE VERFAHREN FÜR DEREN HERSTELLUNG
COLORANTS EN POUDRE ET PROCEDES DE PREPARATION

(30) Priority: 29.10.1990 US 604311
(43) Date of publication of application: 18.08.1993
(73) Proprietor: EASTMAN CHEMICAL COMPANY, Kingsport, TN 37660 (US)
(72) Inventor: KRUTAK, James, John, Sr., Kingsport, TN 37663 (US); PARHAM, William, Whitfield, Kingsport, TN 37664 (US); DARNELL, William, Ronald, Weber City, VA 24251 (US); HENRY, James, Wiliam, Kingsport, TN 37664 (US); OLDFIELD, Terry, Ann, Kingsport, TN 37660 (US)
(74) Representative: Behrens, Dieter, Dr.-Ing.
(86) International application number: US9107854
(87) International publication number: WO9207913

(56) References cited:
- EP-A- 0 040 139
- EP-A- 0 417 017
- WO-A-89/07118
- WO-A-89/10349
- WO-A-91/10693
- DE-A- 3 005 223
- US-A- 4 808 677

## Description

### Field of the Invention

This invention belongs to the field of polymer chemistry. More particularly, this invention relates to polyester powder colorant compositions and a process for their preparation.

### Background of the Invention

Plastics, paints, printing inks, rubber, cosmetics and similar materials typically are colored by organic pigments when superior brilliance and tinctorial strength are important. Toxicity considerations have been a chronic problem relative to the use of organic dyes and pigments since some have been shown to be potential carcinogens and to cause contact dermatitis. Plastics usually contain various additives such as fillers, plasticizers, colorants, etc. The polymeric base of such plastics normally does not produce allergic or other adverse reactions by themselves but leachable or extractable additives are known [Fregert, Manual of Contact Dermatitis, Munksgard Denmark (2nd Ed. 1981)] to cause contact dermatitis.

Various processes for the manufacture of finely-divided forms of polyesters have been disclosed in the prior art such as U.S. Patents 4,378,228, 4,254,207, 3,586,654, 3,931,082, 4,267,310, 4,305,864, 4,451,606, 3,674,736 and 3,669,922. Some of these known processes include the presence of pigments such as carbon black during particle size reduction to produce colored polyester powders. The known procedures are summarized below.
1. Comminution, as by grinding, which is difficult and expensive and results in highly irregular-shaped particles having a broad range of particle size distribution.
2. Spray drying techniques which tend to produce "hollow shells" or porous particles and also are hazardous when organic solvents are used to dissolve the polyester.
3. Dispersion processes which involve melting the polymer in an inert solvent in the presence of a non-ionic dispersing agent. Polyester, in contrast to other thermoplastic polymers, tends to hydrolyze (decompose) when melted in the presence of water and the particles thus produced have a strong tendency to agglomerate or coalesce.
4. Heating under shearing agitation conditions a condensation polymer in an aprotic liquid which is not a solvent for the polymer and in the presence of a dispersing agent to form small liquid particles upon cooling with agitation. Colorants added during this process are still extractable, sublimable, and may exude from the polymer.
5. Solvent induced crystallization wherein an amorphous polymer is initially contacted with a crystal-inducing fluid under certain conditions while the polymer is subjected to physical and/or ultrasonic forces. Colorants added during this process are not reacted with the polymer and therefore are subject to removal from the polymer.
6. Producing microcrystalline polyesters by a hydrolytic removal of amorphous regions of synthetic, linear polyesters followed by a mechanical disintegration of the resulting aggregated microcrystals.
7. Crystallization of polyesters in the presence of nucleating agents.
However, the prior art does not disclose the preparation of colored microcrystalline polyester powders wherein an amorphous or partially-crystalline polyester, having a thermally-stable, colorant compound copolymerized therein, is converted to a colored, microcrystalline, polyester powder by means of a dissolution-crystallization-precipitation procedure. The prior art also fails to disclose microcrystalline, polyester powders containing high levels of colorant incorporated therein which cannot be removed by extraction or sublimation and which does not exude from the surface of the polymer.

It is known that thermoplastic polymeric materials may be colored using color concentrates consisting of physical admixtures of polymers and colorants. However, the use of such physical admixtures to color polymeric materials such as polyesters, e.g., poly(ethylene terephthalate) and blends thereof, presents a number of problems:
(1) Colorant migration during drying of the colored polymer pellets.
(2) Colorant migration during extrusion and colorant accumulation on dies which can cause film rupture and shut-downs for clean-up, etc. Such colorant migration and accumulation result in time consuming and difficult clean-up when a polymer of another color is subsequently processed in the same equipment.
(3) Colorants may not mix well, for example, when using two or more color concentrates to obtain a particular shade.
(4) Colorants may diffuse or exude during storage of the colored polymeric material.
Further, the presence of oligomeric material in the polymers, such as polyester, admixed with the colorants to produce the known color concentrates can cause problems of equipment contamination during processing.

Application WO 91/10693 describes copolymerized colorant-polyester concentrates ; these concentrates, however, contain only methine compounds as colorants.

### Summary of the Invention

This invention pertains to novel, powder colorant compositions comprising a semicrystalline polyester, in a finely-divided form, having copolymerized therein one or more colorant compounds at levels greater than about 1 weight percent. This invention also pertains to a process for the preparation of such colorant compositions comprising dissolution of an amorphous or partially-crystalline polyester color concentrate in a crystallization-inducing solvent or mixture of solvents followed by crystallization or crystallization-precipitation. The colorant compositions have utility in a variety of end uses where nonextractability of the colorant is important.

### Detailed Description of the Invention

The powder colorant compositions provided by this invention comprise a semicrystalline polyester, in a finely-divided form, having copolymerized therein at least 1, more typically at least 5, weight percent, based on the total weight of the compositions, of the residue of a thermally-stable, difunctional colorant compound. Since the powder compositions have colorant residues incorporated into the polymer chain, the colorant is not leachable, sublimable or extractable and does not exude from the composition. The colorant compositions are in the form of a finely-divided, colored microcrystalline polyester powder capable of being used in a wide variety of products such as cosmetics, skin creams or lotions, soaps, hair colorations, waxes, polishes, coatings, paints, toners for impactless printing, inks, etc., which will be safe to humans since exposure to toxic molecules readily absorbed by the body is greatly minimized. Thus, the colorant compositions have utility in a wide variety of applications where toxicological concerns are evident. The concentrate materials may be used for imparting color to a wide variety of thermoplastic compositions including polyesters, polycarbonates, polyamides, cellulose esters, polyurethanes, polyolefins, etc., by conventional melt or solution blending techniques. When using the polymeric color concentrates of this invention, the colorant problems relative to toxicity concerns are largely overcome.

The powder colorant compositions provided by this invention may be obtained by means of a dissolution-crystallization-precipitation procedure wherein a polyester color concentrate, preferably an amorphous or partially crystalline polyester color concentrate, is dissolved in an organic solvent from which the colorant composition is recovered in a finely divided form consisting of particles of relatively uniform size, e.g., from about 10 to 50 microns.

The color concentrates which may be used in the preparation of the powder colorant compositions of the present invention comprise crystalline, semi-crystalline and amorphous polyesters having copolymerized therein at least 1.0, preferably at least 5.0, weight percent of the residues of at least one thermally-stable, difunctional colorant compound. The concentration of the colorant residue in the polyester is dependent on such factors as the end use for which a particular concentrate is designed, the polyester being used, and the physical characteristics required of the color concentrate. Normally, the color concentrates will not contain more than about 50 weight percent of colorant residues with a concentration in the range of about 10 to 40 weight percent being more common. Typically, the polyester color concentrates have an inherent viscosity of at least 0.20 and are comprised of (i) a diacid component consisting of the residues of one or more dicarboxylic acids, (ii) a diol component consisting of the residues of one or more diols and (iii) a colorant component consisting of the residues of one or more thermally-stable, difunctional colorant compounds. The concentration of colorant component (iii) and inherent viscosity are interrelated to the extent that the degree of polymerization and the inherent viscosity should be sufficiently high to ensure that substantially all of the colorant compound is reacted into the polymer and, preferably, into polymer chains which are not extractable. Thus, for example, when the concentration of colorant component (iii) is 20 weight percent or higher, the inherent viscosity of the polyester normally will be about 0.25 or higher.

The diacid residues may be derived from aliphatic, alicyclic, or aromatic dicarboxylic acids such as terephthalic acid, isophthalic acid, 1,4-cyclohexanedicarboxylic acid, 1,3-cyclohexanedicarboxylic acid, succinic acid, glutaric acid, adipic acid, sebacic acid, 1,12-dodecanedioic acid, 2,6-naphthalenedicarboxylic acid and the like. In the polymer preparation, it is often preferable to derive the diacid residues from an ester-forming derivative of the dicarboxylic acid such as the dimethyl, diethyl, or dipropyl esters. The anhydrides or acid halides of these acids also may be employed where practical.

The diol components of the described polyesters may be selected from ethylene glycol, 1,2-propanediol, 1,3-propanediol, 2-methyl-1,3-propanediol, 1,4-butanediol, 2,2-dimethyl-1,3-propanediol, 1,6-hexanediol, 1,10-decanediol, 1,12-dodecanediol, 1,2-cyclohexanediol, 1,4-cyclohexanediol, 1,2-cyclohexanedimethanol, 1,3-cyclohexanedimethanol, 1,4-cyclohexanedimethanol, 2,2,4,4-tetramethyl-1,3-cyclobutanediol, X,8-bis-(hydroxymethyl)-tricyclo-[5.2.1.0]-decane wherein X represents 3, 4, or 5; and diols containing one or more oxygen atoms in the chain, e.g., diethylene glycol, triethylene glycol, dipropylene glycol, tripropylene glycol, 1,3- and 1,4-bis(2-hydroxyethyl)benzene and the like. In general, these diols contain 2 to 18, preferably 2 to 12 carbon atoms. Cycloaliphatic diols can be employed in their cis or trans configuration or as mixtures of both forms.

The preferred amorphous color concentrates from which our novel powder colorant compositions may be prepared exhibit a glass transition temperature (Tg) and no, or only a trace of, crystallization or melting point by differential scanning calorimetry (DSC). Examples of such amorphous polyesters include those obtained by the polymerization of a difunctional colorant compound, terephthalic and/or 2,6-naphthalenedicarboxylic acid and a branched-chain diol having the formula 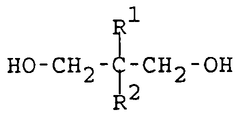
wherein R¹ is hydrogen or an unsubstituted or substituted alkyl, cycloalkyl or aryl radical, and R² is an unsubstituted or substituted alkyl, cycloalkyl or aryl radical. Preferred amorphous polyester color concentrates have an inherent viscosity of about 0.2 to 0.8 and are comprised of:
(i) diacid residues comprised of at least 50, preferably at least 80, mole percent terephthalic and/or 2,6-naphthalenedicarboxylic acid residues;
(ii) diol residues comprised of at least 50, preferably at least 80, mole percent of residues of a diol having the formula 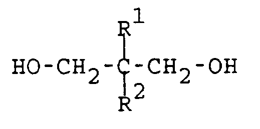 wherein R¹ is hydrogen or lower alkyl and R² is lower alkyl; and
(iii) residues of a thermally-stable, difunctional colorant compound.
The particularly preferred amorphous polyester color concentrates are comprised of (i) diacid residues consisting essentially of terephthalic and/or 2,6-naphthalenedicarboxylic acid residues; (ii) diol residues consisting essentially of 2,2-dimethyl-1,3-propanediol residues; and (iii) residues of one or more colorant compounds.

Other amorphous polyesters, as defined above, suitable for preparing the powder colorant compositions may be obtained by employing (1) two dicarboxylic acids and one or more diols or (2) two diols and one or more dicarboxylic acids according to known procedures for obtaining amorphous polyesters. The polyester comprising a diacid component consisting of 75 mole percent terephthalic acid residues and 25 mole percent 1,4-cyclohexanedicarboxylic acid residues, a diol component comprised of 1,4-butanediol residues and residues of a difunctional colorant compound of Formula (I) is an example of such a polyester.

The partially-crystalline color concentrates useful in the preparation of the powder colorant compositions of this invention usually exhibit a glass transition temperature, a crystallization temperature and a melting temperature by DSC. These partially-crystalline, polyester concentrates are comprised of (i) diacid residues consisting of at least 80 mole percent terephthalic acid residues, 2,6-naphthalenedicarboxylic acid residues, 1,3-cyclohexanedicarboxylic acid residues, 1,4-cyclohexanedicarboxylic acid residues or a mixture thereof, (ii) diol residues comprised of at least 50 mole percent of residues having the formula -O-(CH₂)ₚ-O-wherein p is 2, preferably 4, to 12 and (iii) residues of a thermally-stable, difunctional, colorant compound. A preferred partially-crystalline color concentrate has a melting temperature of at least 110°C and is comprised of (i) diacid residues comprised of at least 80 mole percent terephthalic acid residues, (ii) diol residues comprised of at least 80 mole percent of residues of 1,4-butanediol and (iii) residues of the colorant compound. An especially preferred partially-crystalline color concentrate has a melting temperature of at least 110°C and consists essentially of (i) terephthalic acid residues, (ii) 1,4-butanediol residues and (iii) residues of one of the difunctional, colorant compounds described hereinbelow.

The polyester color concentrates may be prepared according to conventional esterification or transesterification and melt polycondensation procedures using (i) a dicarboxylic acid or, preferably, a lower alkyl ester thereof, (ii) a diol and (iii) a thermally stable colorant compound bearing two reactive groups. Normally, a 50 mol percent excess of the diol is used. The colorant compound preferably is added with the other monomers at the commencement of the color concentrate manufacture although it may be added subsequently, e.g., at the beginning or during the polycondensation step. The concentration (weight percent) of the colorant residue is determined by summing up the weights of all the components charged to the reactor and subtracting the sum of the weights of the components removed during transesterification and polycondensation, e.g., methanol and excess diol. The difference represents the theoretical yield of the color concentrate. The weight of the colorant charged to the reactor is divided by the theoretical weight and multiplied by 100 to give the weight percent of colorant residue.

The thermally-stable, difunctional, colorant compounds useful in the preparation of the polyester color concentrates and, thus, the powder colorant compositions of the present invention may be selected from a wide variety of colorant compounds of diverse structure types and/or classes. The only requirements of useful colorant compounds is that (1) they are stable under polyester polymerization conditions and (2) their structures bear at least two polyester-reactive substituents. Examples of the types or classes of colorant compounds which may be used include methines, bis-methines, anthraquinones, 2,5-diarylaminoterephthalic acids, quinophthalones, thioxanthones, 3H-dibenz[f,ij] isoquinoline-2,7-diones (anthrapyridones), 7H-dibenz[f,ij]isoquinoline-7-ones (anthrapyridines), 7H-benz[e]perimidine-7-ones, 7-amino-2H-1-benzopyran-2-ones (coumarins), triphenodioxazines, 5,12-dihydroquinoxalino [2,3-b]phenazines (fluorindines), phthaloylpyrrocolines, 4-aminonaphthalimides, 3,6-diaminopyromellitic acid dimides, benzanthrones, naphtho[1',2',3':4,5]quino[2,1-b]quinazoline-5,10-diones, 6,15-dihydro-5,9,14,18-anthrazinetetrones (indanthrones), 5H-benzo[a]phenoxazine-5-ones, 6H,13H-pyrido[1,2-a:3,4-b] diindole-6,13-diones, diindolo[3,2,1-de-3',2',1'-ij][1,5] naphthyridine-6,13-diones, perylenes, perinones, napthalene-1,4,5,8-tetracarboxylic acids and diimides, quinacridones and phthalocyanines. Specific examples of suitable colorant compounds are described in U.S. Patents 3,417,048, 3,489,713, 4,080,355, 4,088,650, 4,049,376, 4,116,923, 4,267,306, 4,359,570, 4,403,092, 4,594,400, 4,740,581, 4,745,173, 4,617,373, 4,804,719, 4,808,677 and 4,892,922, and British Patent 1,225,566.

The colorant compounds described above may be represented by the formula

Col-(X)₂ (I)

wherein Col is the residue of one of the types of colorants set forth above and X is a polyester-reactive substituent, i.e., a group reactive with at least one of the monomers from which the polyester is prepared. Examples of the reactive groups which X may represent include hydroxy, carboxy, an ester radical, amino, alkylamino, and the like. The ester radicals may be any radical having the formula 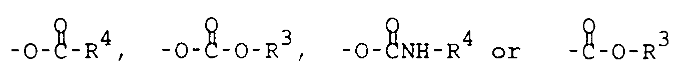
wherein R³ is selected from unsubstituted or substituted alkyl, cycloalkyl or aryl radicals. R³ preferably is unsubstituted alkyl, e.g., alkyl of up to about 8 carbon atoms, or phenyl, and most preferably, lower alkyl, e.g., methyl and ethyl. R⁴ is hydrogen or selected from those groups listed for R³. Reactive group X preferably is hydroxy, carboxy, carbalkoxy, or alkanoyloxy of up to about 4 carbon atoms, e.g., carbomethoxy or acetoxy. Known colorant compounds may, if necessary, be functionalized by the addition thereto of reactive groups represented by X according to known procedures.

The powder colorant compositions described hereinabove may be prepared in accordance with the present invention by a dissolution-crystallization-precipitation process comprising the steps of:
(1) dissolving in an inert, organic solvent a polyester color concentrate comprising a crystalline or, preferably, a partially crystalline or amorphous polyesters having copolymerized therein at least 1.0, preferably at least 5.0, weight percent of the residues of at least one thermally-stable, difunctional colorant compound, e.g., a compound of formula (I); and
(2) precipitating from the solution of step (1) the semicrystalline colorant composition in a finely divided form consisting of particles of relatively uniform size, e.g., from about 10 to 50 microns.
If desired, the particle size of the powder colorant compositions obtained from the process may be reduced further by conventional grinding processes.

Typically, step (1) is carried out using a solvent in which the polyester color concentrate is soluble to the extent of at least 100 g concentrate per liter of inert solvent. Examples of solvents in which the amorphous and/or partially-crystalline concentrates may be dissolved include halogenated hydrocarbons such as aliphatic chlorides, e.g., methylene chloride; alkyl carboxylic acid esters containing 3 to about 10 carbon atoms, e.g., ethyl acetate and methyl benzoate; hydrocarbons such as toluene; and ethers such as tetrahydrofuran. We have found methylene chloride to be a particularly effective solvent.

The amount of solvent required may vary substantially depending on the particular inert solvent or combination of inert solvents used, the particular polyester color concentrate used and the temperature at which the dissolution is carried out. Typically, the ratio of the concentrate:solvent (weight:weight) is in the range of about 1:3 to 1:15, preferably about 1:4 to 1:8. The temperature at which the dissolution step is performed may be in the range of about 25°C up to the boiling point of the solvent.

The second step of the process may be accomplished by cooling the step (1) solution, with or without a reduction in the volume of solvent, i.e., either with or without a solution concentration step. Another useful technique involves adding to the step (1) solution a miscible, inert, organic liquid/solvent (usually having a higher boiling point) which causes crystallization and precipitation of the colored semicrystalline powder, either with or without partial, or essentially complete, removal of the step (1) solvent by distillation. The use of methylene chloride as the step (1) solvent and an alkyl acetate such as ethyl acetate as the "crystallization-inducing" solvent has been found to be particularly efficacious and preferred.

Depending on their intended utility, the powder colorant compositions obtained in accordance with our novel process may be extracted with a suitable organic solvent to remove relatively low molecular weight polyester oligomers. Examples of oligomer-extracting solvents include ketones such as acetone, 2-pentanone, 3-methyl-2-butanone, 4-methyl-2-pentanone, 2-hexanone and 5-methyl-2-hexanone; hydrocarbons such as hexane, heptane and toluene; and ethers such as tetrahydrofuran. Another, but not preferred, dissolution-precipitation procedure involves dissolving an amorphous color concentrate in certain solvents, e.g., ethyl acetate, from which the polymeric color concentrate, after undergoing a change in morphology, precipitates.

Some of the more crystalline polyesters such as poly(ethylene terephthalate) and poly(tetramethylene terephthalate) require the use of a high-boiling solvent in the dissolution-precipitation procedure. Examples of such high-boiling solvents include alkyl esters of aromatic mono- and di-carboxylic acids, e.g. alkyl benzoates and alkyl phthalates; aliphatic dicarboxylic acid esters; glycol esters, e.g. ethylene glycol diacetate; diethylene glycol diacetate; aromatic ketones, e.g., acetophenone; aromatic oxides, e.g. diphenyl oxide; aliphatic carboxamides, e.g. N,N-dimethylformamide; isophorone. Methyl benzoate and ethylene glycol diacetate are particularly preferred high-boiling solvents since they are readily available, have a pleasant odor and do not cause color problems during crystallization which sometimes is a problem with the aromatic ketones.

In one embodiment of the process of our invention, a crude polyester color concentrate is prepared and granulated to a very coarse powder which is then heated with a high-boiling solvent, e.g. methyl benzoate, to facilitate dissolution. Upon cooling, crystallization-precipitation occurs and a diluent such as acetone usually is needed to permit stirring. Filtration gives a finely-divided powder which may require washing or reslurrying to remove the crystallization solvent and low molecular weight oligomeric material.

In another variation of the dissolution-crystallization-precipitation process, crystallization can occur as an integral part of the polyester color concentrate manufacturing process wherein a high-boiling solvent crystallization solvent is added to a melt of the concentrate to obtain a solution of the color concentrate which then may be obtained as a powder by precipitation. The polyester color concentrate powder is thus obtained in a purified form without the need of a granulation step by a means which may be used in conjunction with batch processing. The solvent used in this embodiment normally should have a boiling point of at least 120°C, preferably in the range of about 150 to 275°C. Examples of such high-boiling inert solvents include lower alkyl, e.g., alkyl of up to about 4 carbon atoms, esters of aromatic mono- and di-carboxylic acids such as methyl benzoate, butyl benzoate, dimethyl phthalate; glycol esters, e.g., ethylene glycol diacetate; aromatic ethers such as diphenyl oxide; alkanoyl benzenes, such as acetophenone; and glycol ethers.

The dissolution-crystallization-precipitation procedure alters the morphology of the amorphous and partially-crystalline polyester color concentrates in a number of respects. X-Ray diffraction analysis of the colored semicrystalline powders shows a marked increase in the crystallinity of the polyester and, while the amorphous polyester concentrates do not exhibit a melting temperature, the microcrystalline concentrates usually (almost always) exhibit a melting temperature by DSC. Although the weight average molecular weight (Mw) may either increase or not be changed by the dissolution-crystallization-precipitation procedure, the number average molecular weight (Mn) always increases, the magnitude of the increase depending on the degree to which oligomeric material has been removed from the colored semicrystalline polyester powder. The polydispersity ratio (Mw:Mn) of the colored semicrystalline polyester is always less than that of the polyester concentrate from which it is prepared due to the increase in Mn (even when Mw increases, Mn increases more). Finally, the inherent viscosity of the colored semicrystalline powders normally is slightly higher than that of the corresponding color concentrates.

A multiplicity of colors of semicrystalline polyester powders may be obtained by combining individual colors, e.g., subtractive colors such as yellow, magenta and cyan according to known color technology (see N. Ohta, Photographic Science and Engineering, Volume 15, No. 5, Sept.-Oct. 1971, pp. 399-415). In the practice of this invention the colors may be combined at various appropriate stages in the preparation of the semicrystalline powders:
(a) two or more copolymerizable colorants are added to the initial polymerization reaction; upon completion of the polycondensation reaction the colored semicrystalline powder is prepared via the above-mentioned dissolution-crystallization-precipitation procedure;
(b) two or more colored amorphous or partially crystalline color concentrates are combined and then converted to a colored semi-crystalline polyester powder via the dissolution-crystallization-precipitation;
(c) two or more colored semicrystalline polyester powders are combined and the dissolution-crystallization-precipitation procedure repeated;
(d) two or more colored semicrystalline polyester powders are physically admixed by using known blending methods.
Further, the polyester compositions of the present invention may possess one or more difunctional colorants or may exist as a mixture of polyester compositions, each possessing one or more difunctional colorants. Also, as well understood by those skilled in the art, conventional additives may be present in the colored semicrystalline polyester powder composition of the present invention. For instance, such additives may include plasticizers, flame retardants, nucleating agents, stabilizers, antioxidants or opacifiers such as titanium dioxide.

Our novel process and the powder colorant compositions obtained therefrom are further illustrated by the following examples. The inherent viscosities specified herein are determined at 25°C using 0.5 g of polymer (polyester color concentrate or powder colorant composition) per 100 mL of a solvent consisting of 60 weight percent phenol and 40 weight percent tetrachloroethane. The weight average molecular weight (Mw) and number average molecular weight (Mn) values referred to herein are determined by gel permeation chromatography. The melting temperatures are determined by differential scanning calorimetry on the first and/or second heating cycle at a scanning rate of 20°C per minute and are reported as the peaks of the transitions.

### EXPERIMENTAL SECTION

### EXAMPLE 1

The following materials are placed in a 500 mL, three-necked, round-bottom flask:
110.4 g (0.569 mol) dimethyl terephthalate
70.5 g (1.138 mol) ethylene glycol
0.0119 g Ti from a n-butanol solution of titanium isopropoxide
12.07 g (0.0215 mol) N,N'-bis-(2,2-dimethyl-3-hydroxypropyl)-3,4,9,10-perylene-tetracarboxylic diimide
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 75 minutes. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for about 45 minutes under a pressure of 0.1 to 0.5 mm Hg. The vacuum is relieved with nitrogen and methyl benzoate (175 mL) is added slowly. The mixture is stirred to solution over about 10 minutes with the flask still in the metal bath. The heat is then removed and stirring continued. Crystallization begins to occur at about 115°C. At 50°C, acetone (150 mL) is added to facilitate stirring. The diluted slurry is stirred for about 30 minutes, filtered and the cake washed three times with acetone and dried. The resulting dark red polyester contains 10.1 weight percent of the perylene colorant residue, has an inherent viscosity of 0.44, a weight average molecular weight of 43,741, a number average molecular weight of 17,837 and a polydispersity value of 2.45. The yield of powder is 111 g.

### EXAMPLE 2

The following materials are placed in a 500 mL, three-necked, round-bottom flask:
80.44 g (0.415 mol) dimethyl terephthalate
67.67 g (0.752 mol) 1,4-butanediol
0.0137 g Ti from a n-butanol solution of titanium tetraisopropoxide
41.42 g (0.0866 mol) 1,5-bis(2-carboxyanilino)anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for 5 minutes under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Acetone:hexane (1:1 by volume) mixture (1.0 L) is added slowly with stirring to dilute the slurry and keep it stirrable. The diluted slurry is stirred for 30 minutes, filtered and the cake is washed with acetone:hexane (1:1) mixture. The cake is twice reslurried in acetone and then dried in air. The resulting dark red semicrystalline polyester powder, containing 30.16 weight percent of the anthraquinone colorant residue, has an inherent viscosity of 0.144, a melting temperature of 195°C, a weight average molecular weight of 9,828, a number average molecular weight of 7,037 and a polydispersity value of 1.39. The weight of recovered powder is 127 g.

### EXAMPLE 3

The following materials are placed in a 500 mL, three-necked, round-bottom flask:
81.38 g (0.419 mol) dimethyl terephthalate
68.00 g (0.755 mol) 1,4-butanediol
0.0139 g Ti from a n-butanol solution of titanium tetraisopropoxide
43.10 g (0.0842 mol) 1,5-bis(o-carboxyphenylthio)anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for 5 minutes under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Acetone:hexane (1:1 by volume) mixture (1.0 L) is added slowly with stirring to dilute the slurry and keep it stirrable. The diluted slurry is stirred for 30 minutes, filtered and the cake is washed with acetone:hexane (1:1) mixture. The cake is twice reslurried in acetone and then dried in air. The resulting yellow semicrystalline polyester powder, containing 30.79 weight percent of the anthraquinone colorant residue, has an inherent viscosity of 0.12, a melting temperature of 178°C, a weight average molecular weight of 8,500, a number average molecular weight of 6,477 and a polydispersity value of 1.31. The weight of recovered powder is 122 g.

### EXAMPLE 4

The following materials are placed in a 500 mL, three-necked, round-bottom flask:
58.20 g (0.30 mol) dimethyl terephthalate
40.50 g (0.45 mol) 1,4-butanediol
0.0025 g Ti from a n-butanol solution of titanium tetraisopropoxide
22.0 g (0.029 mol) blue anthraquinone colorant having the formula: 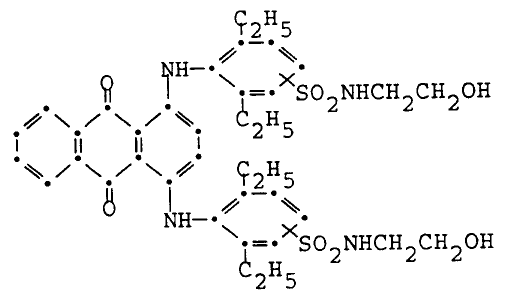
The flask is equipped with a nitrogen inlet, stirrer, vacuum outlet, and condensing flask. The flask and contents are heated in a Belmont metal bath with a nitrogen sweep over the reaction mixture as the temperature is increased to 200° and then to 220° over 2 hours. Over the next 30 minutes the temperature is increased to about 240° and then to about 260° over the next 30 minutes. The temperature is quickly raised (over about 10 minutes) to 275° and a vacuum is applied until the pressure is reduced to 0.5 mm Hg. The polycondensation is completed by heating the flask and contents at about 275° for 5 minutes under a pressure of 0.1 to 0.5 mm Hg. The vacuum is then relieved with nitrogen and methyl benzoate (100 mL) is added slowly and stirred to solution over about 10 minutes with the flask still in the metal bath. The resulting solution is transferred to a 2 L beaker and stirred until crystallization occurs. Hexane (700 mL) is added slowly with stirring to dilute the slurry and keep it stirrable. The diluted slurry is stirred for 30 minutes, filtered and the cake is washed with hexane three times and then dried in air. The resulting dark blue semicrystalline polyester powder, containing 25.76 weight percent of the anthraquinone colorant residue, has an inherent viscosity of 0.103, a melting temperature of 182°C, a weight average molecular weight of 4,181, a number average molecular weight of 1,855 and a polydispersity value of 2.25. The weight of recovered powder is 74.4 g.

### EXAMPLE 5

The following materials are placed in a 500 mL, three-necked, round-bottom flask:
64.22 g (0.331 mol) dimethyl terephthalate
48.89 g ((0.543 mol) 1,4-butanediol
0.0099 g Ti from a n-butanol solution of titanium tetraisopropoxide
30.0 g (0.0619 mol) red anthrapyridone colorant having the formula 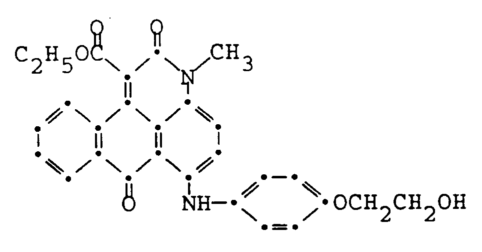
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for 5 minutes under a pressure of 0.1 to 0.5 mm Hg. The vacuum is then relieved with nitrogen and methyl benzoate (80 mL) is added slowly and stirred to solution over about 10 minutes with the flask still in the metal bath. The resulting solution is transferred to a 2 L beaker and stirred until crystallization occurs. Acetone (1.0 L) is added slowly with stirring to dilute the slurry and keep it stirrable. The diluted slurry is stirred for 30 minutes, filtered and the cake is washed with acetone. The cake is twice reslurried in acetone and then dried in air. The resulting dark red semicrystalline polyester powder, containing 29.99 weight percent of the anthrapyridone colorant residue, has an inherent viscosity of 0.134, a melting temperature of 199°C, a weight average molecular weight of 7,531, a number average molecular weight of 5,258 and a polydispersity value of 1.43. The weight of recovered powder is 91.2 g.

### EXAMPLE 6

The following materials re placed in a 500 mL, three-necked, round-bottom flask:
97.00 g (0.50 mol) dimethyl terephthalate
67.50 g (0.75 mol) 1,4-butanediol
0.0115 g Ti from a n-butanol solution of titanium tetraisopropoxide
6.0 g (.0106 mol) N,N'-bis-(2,2-dimethyl-3-hydroxypropyl)-3,4,9,10-perylenetetracarboxylic diimide
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 225°C over 2 hours. Over the next 50 minutes the temperature is increased to about 255°C. The temperature is quickly raised (over about 25 minutes) to 270°C and a vacuum is applied until the pressure is reduced to 0.5 mm Hg. The polycondensation is completed by heating the flask and contents at about 270°C for 15 minutes under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Acetone (700 mL) is added slowly with stirring to dilute the slurry and keep it stirrable. The diluted slurry is stirred for 30 minutes, filtered and the cake is washed with acetone. The cake is twice reslurried in acetone and then dried in air. The resulting dark red semicrystalline polyester powder, containing 5.21 weight percent of the perylene colorant residue, has an inherent viscosity of 0.418, a melting temperature of 224°C, a weight average molecular weight of 40,954, a number average molecular weight of 18,337 and a polydispersity value of 2.23. The weight of recovered powder is 110 g.

### EXAMPLE 7

The following materials are placed in a 500 mL, three-necked, round-bottom flask:
75.44 g (0.388 mol) dimethyl terephthalate
52.50 g (0.583 mol) 1,4-butanediol
0.0115 g Ti from a n-butanol solution of titanium tetraisopropoxide
36.10 g (0.0736 mol) red anthraquinone colorant having the formula: 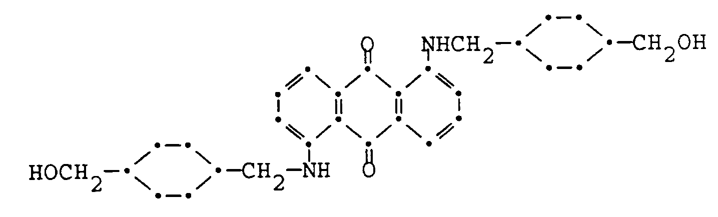
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 225°C over 2.5 hours. A vacuum is applied until the pressure is reduced to 0.5 mm Hg. The polycondensation is completed by heating the flask and contents at about 225°C for 1 hour under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Hexane (700 mL) is added slowly with stirring to dilute the slurry and keep it stirrable. The diluted slurry is stirred for 30 minutes, filtered and the cake is washed with hexane. The cake is twice reslurried in hexane and then dried in air. The resulting dark red semicrystalline polyester powder, containing 31.38 weight percent of the anthraquinone colorant residue, has an inherent viscosity of 0.397, a melting temperature of 188°C, a weight average molecular weight of 21,296, a number average molecular weight of 12,060 and a polydispersity value of 1.76. The yield of powder is 85.3 g.

### EXAMPLE 8

The following materials are placed in a 500 mL, three-necked, round-bottom flask:
135.80 g (0.70 mol) dimethyl terephthalate
94.50 g (1.05 mol) 1,4-butanediol
0.0208 g Ti from a n-butanol solution of titanium tetraisopropoxide
62.0 g (0.083 mol) blue anthraquinone colorant used in Example 4
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 225°C over 2.5 hours. A vacuum is applied until the pressure is reduced to 0.5 mm Hg. The polycondensation is completed by heating the flask and contents at about 225°C for 1 hour under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Hexane (800 mL) is added slowly with stirring to dilute the slurry and keep it stirrable. The diluted slurry is stirred for 30 minutes, filtered and the cake is washed with hexane. The cake is twice reslurried in hexane and then dried in air. The resulting dark blue semicrystalline polyester powder, containing 29.72 weight percent of the anthraquinone colorant residue, has an inherent viscosity of 0.167, a melting temperature of 182°C, a weight average molecular weight of 8,520, a number average molecular weight of 5,372 and a polydispersity value of 1.59. The yield of powder is 199 g.

### PREPARATION 1

The following materials are placed in a 500 mL, three-necked, round-bottom flask:
135.8 g (0.70 mol) dimethyl terephthalate
94.6 g (0.91 mol) 2,2-dimethyl-1,3-propanediol
0.0170 g Ti from a n-butanol solution of titanium tetraisopropoxide
11.73 g (0.024 mol) red methine colorant having the structure 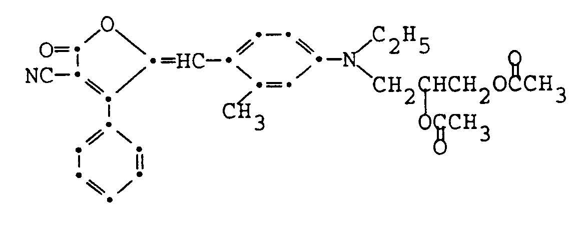
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 30 minutes the temperature is increased to about 240°C and then to about 260°C over the next 30 minutes. The temperature is quickly raised (over about 10 minutes) to 275°C. With a stream of nitrogen bleeding into the system a vacuum is applied until the pressure is reduced to about 0.5 mm Hg. The polycondensation is completed by heating the flask and contents at about 275°C for about 45 minutes under a pressure of 0.1 to 0.5 mm Hg. The flask is removed from the metal bath and is allowed to cool while the polymer solidifies. The resulting high molecular weight red polyester, containing 7.0 weight percent of the methine colorant residue, has an inherent viscosity of 0.57, no melting temperature, a weight average molecular weight of 45,428, a number average molecular weight of 9,349 and a polydispersity value of 4.86.

### EXAMPLE 9

A portion (50.0 g) of the amorphous color concentrate prepared in PREPARATION 1 is granulated using a Wiley mill and added portionwise to toluene (200 mL) stirred in an explosion-proof Waring blender. After complete addition, stirring is continued at full speed for about 20 minutes and with the temperature rising to about 80°C. Additional toluene is added to rinse down the walls of the blender container and the mixture allowed to stand overnight to produce solid semicrystalline material. The volume of the mixture is doubled by the addition of acetone. The solid product is collected by filtration and then reslurried four times in acetone by stirring in the Waring blender followed by filtration after each reslurry. After drying in air the red semi-crystalline polyester powder weighs 46.0 g, and has an inherent viscosity of 0.58, a weight average molecular weight of 45,167, a number average molecular weight of 13,919 and a polydispersity value of 3.24.

The above procedure for crystallization of the poly(2,2-dimethyl-1,3-propanediyl terephthalate) color concentrates is used, with minor modifications, in Examples 10, 11, 12, 13, 14, 15 and 16. Depending upon the solubility of the amorphous color concentrate, stirring is usually continued at full speed for 20-30 minutes or until solution occurs or until the temperature approaches the boiling point of toluene.

### EXAMPLE 10

The following materials are placed in a 500 mL, three-necked, round-bottom flask:
129.2 g (0.666 mol) dimethyl terephthalate
94.6 g (0.91 mol) 2,2-dimethyl-1,3-propanediol
0.01744 g Ti from a n-butanol solution of titanium tetraisopropoxide
16.4 g (0.0343 m) 1,5-bis(2-carboxyanilino)anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to about 0.5 mm Hg. The polycondensation is completed by heating the flask and contents at about 275°C for about 1 hour under a pressure of 0.1 to 0.5 mm Hg. The flask is removed from the metal bath and is allowed to cool while the polymer solidifies. The resulting high molecular weight, red polyester, containing 9.39 weight percent of the anthraquinone colorant residue, has an inherent viscosity of 0.53, no melting temperature, a weight average molecular weight of 33,986, a number average molecular weight of 9,805 and a polydispersity value of 3.47.

A portion (50.0 g) of the amorphous color concentrate is converted into a colored semicrystalline polyester powder using the general procedure described in Example 9. The yield is 45.3 g of red powder having an inherent viscosity of 0.55, a melting temperature of 138°C, a weight average molecular weight of 44,436, a number average molecular weight of 16,591 and a polydispersity value of 2.67.

### EXAMPLE 11

The following materials are placed in a 500 mL, three-necked, round-bottom flask:
135.80 g (0.70 mol) dimethyl terephthalate
94.6 g (0.91 mol) 2,2-dimethyl-1,3-propanediol
0.01763 g Ti from a n-butanol solution of titanium tetraisopropoxide
16.0 g (0.0355 mol) 1,4-bis[4-(2-hydroxyethyl)anilino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 90 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to 0.5 mm Hg. The polycondensation is completed by heating the flask and contents at about 275°C for about 1 hour under a pressure of 0.1 to 0.5 mm Hg. The flask is removed from the metal bath and is allowed to cool while the polymer solidifies. The resulting high molecular weight, greenish-blue polyester, containing 9.08 weight percent of the anthraquinone colorant residue, has an inherent viscosity of 0.49, no melting temperature, a weight average molecular weight of 38,584, a number average molecular weight of 17,769 and a polydispersity value of 2.17.

A portion (50 g) of the amorphous color concentrate is crystallized using the general described in Example 9 to produce a greenish-blue semicrystalline polyester powder (44.8 g) having an inherent viscosity of 0.50, a melting temperature of 138°C, a weight average molecular weight of 45,185, a number average molecular weight of 17,562 and a polydispersity value of 2.57.

### EXAMPLE 12

The following materials are placed in a 500 mL, three-necked, round-bottom flask:
135.80 g (0.70 mol) dimethyl terephthalate
94.60 g (0.91 mol) 2,2-dimethyl-1,3-propanediol
0.01762 g Ti from a n-butanol solution of titanium tetraisopropoxide
16.0 g (0.0314 mol) 1,4-bis[4-(2-hydroxyethoxy)anilino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to about 0.5 mm Hg. The polycondensation is completed by heating the flask and contents at about 275°C for about 1 hour under a pressure of 0.1 to 0.5 mm Hg. The flask is removed from the metal bath and is allowed to cool while the polymer solidifies. The resulting high molecular weight polyester, containing 9.08 weight percent of the greenish-blue colorant residue, has an inherent viscosity of 0.71, no melting temperature, a weight average molecular weight of 49,918, a number average molecular weight of 23,654 and a polydispersity value of 2.1.

A portion (50.0 g) of the amorphous color concentrate is converted into a colored semicrystalline polyester powder using the general procedure described in Example 9. The yield is 45.6 g of a greenish-blue powder which has a melting temperature of 137°C, an inherent viscosity of 0.72, a weight average molecular weight of 50,959, a number average molecular weight of 30,849 and a polydispersity of 1.44.

### EXAMPLE 13

The following materials are placed in a 500 mL, three-necked, round-bottom flask:
131.3 g (0.677 mol) dimethyl terephthalate
145.6 g (1.40 mol) 2,2-dimethyl-1,3-propanediol
0.01638 g Ti from a n-butanol solution of titanium tetraisopropoxide
11.73 g (0.0229 m) 1,5-bis(2-carboxyphenylthio)anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 15 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to 0.5 mm Hg. The polycondensation is completed by heating the flask and contents at about 275°C for about 1.25 hours under a pressure of 0.1 to 0.5 mm Hg. The flask is removed from the metal bath and is allowed to cool while the polymer solidifies. The resulting high molecular weight yellow polyester, containing 7.16 weight percent of the anthraquinone colorant residue, has an inherent viscosity of 0.57, no melting temperature, a weight average molecular weight of 36,032, a number average molecular weight of 10,391 and a polydispersity value of 3.47. A portion (50 g) of the amorphous polymer is crystallized as in Example 9 to yield 44.3 g of yellow semicrystalline polyester powder.

### EXAMPLE 14

The following materials are placed in a 500 mL, three-necked, round-bottom flask:
131.3 g (0.677 mol) dimethyl terephthalate
145.6 g (1.40 mol) 2,2-dimethyl-1,3-propanediol
0.01638 g Ti from a n-butanol solution of titanium tetraisopropoxide
11.73 g (0.0245 m) 1,5-bis-(2-carboxyanilino)anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to 0.5 mm Hg. The polycondensation is completed by heating the flask and contents at about 275°C for about 1.25 hours under a pressure of 0.1 to 0.5 mm Hg. The flask is removed from the metal bath and is allowed to cool while the polymer solidifies. The resulting high molecular weight red polyester, containing 7.16 weight percent of the anthraquinone colorant residue, has an inherent viscosity of 0.60, no melting temperature, a weight average molecular weight of 35,420, a number average molecular weight of 10,175 and a polydispersity value of 3.48.

A portion (50.0 g) of the amorphous polymer is crystallized according to the general procedure of Example 9 to yield 45.2 g of red semicrystalline polyester powder.

### EXAMPLE 15

The following materials are placed in a 500 mL, three-necked, round-bottom flask:
135.8 g (0.70 mol) dimethyl terephthalate
145.6 g (1.40 mol) 2,2-dimethyl-1,3-propanediol
0.01638 g Ti from a n-butanol solution of titanium tetraisopropoxide
11.73 g (0.0216 m) methine colorant having the structure: 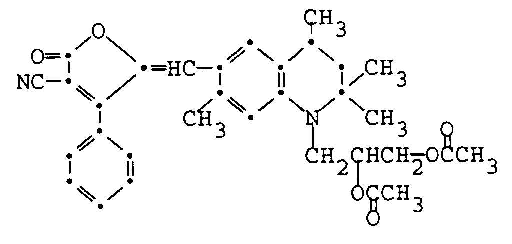
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to 0.5 mm Hg. The polycondensation is completed by heating the flask and contents at about 275°C for about 1.25 hours under a pressure of 0.1 to 0.5 mm Hg. The flask is removed from the metal bath and is allowed to cool while the polymer solidifies. The resulting high molecular weight red polyester, containing 7.16 weight percent of the methine colorant residue, has an inherent viscosity of 0.56, no melting temperature, a weight average molecular weight of 37,995, a number average molecular weight of 9,014 and a polydispersity value of 4.2.

A portion (50 g) of the amorphous polymer is crystallized as in Example 9 to yield 45.4 g of bright magenta, semicrystalline, polyester powder.

### EXAMPLE 16

The following materials are placed in a 500 mL, three-necked, round-bottom flask:
135.8 g (0.70 mol) dimethyl terephthalate
145.6 g (1.40 mol) 2,2-dimethyl-1,3-propanediol
0.01638 g Ti from a n-butanol solution of titanium tetraisopropoxide
11.73 g (0.0239 mol) blue anthraquinone colorant having the structure: 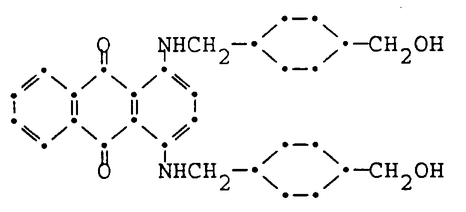
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 30 minutes. Over the next 20 minutes the temperature is increased to about 240°C and then to about 260°C over the next 15 minutes. The temperature is quickly raised (over about 10 minutes) to 275°C. With a stream of nitrogen bleeding into the system a vacuum is applied until the pressure is reduced to 0.5 mm Hg. The polycondensation is completed by heating the flask and contents at about 275°C for abut 1.25 hours under a pressure of 0.1 to 0.5 mm Hg. The flask is removed from the metal bath and is allowed to cool while the polymer solidifies. The resulting high molecular weight dark blue polyester, containing 7.16 weight percent of the anthraquinone colorant residue, has an inherent viscosity of 0.524, no melting temperature, a weight average molecular weight of 33,811, a number average molecular weight of 9,708 and a polydispersity value of 3.48.

A portion (50.0 g) of the amorphous polymer is crystallized as in Example 9 to yield 45.8 g of greenish-blue semicrystalline polyester powder.

### PREPARATION 2

The following materials are placed in a 500-mL, three-necked, round-bottom flask:
133.6 g (0.689 mol) dimethyl terephthalate
85.5 g (1.380 mol) ethylene glycol
0.0178 g Ti from a n-butanol solution of titanium tetraisopropoxide
54.0 g (0.132 mol) 1,5-bis[(3-hydroxy-2,2-dimethylpropyl)amino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 75 minutes. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for about 45 to 60 minutes under a pressure of 0.1 to 0.5 mm Hg. The flask is removed from the metal bath and is allowed to cool while the polymer solidifies. The resulting dark red polyester, containing 30.3 weight percent of the anthraquinone colorant residue, has an inherent viscosity of 0.49.

### PREPARATION 3

The following materials are placed in a 500-mL, three-necked, round-bottom flask:
135.8 g (0.70 mol) dimethyl terephthalate
94.6 g (0.91 mol) 2,2-dimethyl-1,3-propanediol
0.0177 g Ti from a n-butanol solution of titanium tetraisopropoxide
18.0 g (0.044 mol) 1,5-bis[(3-hydroxy-2,2-dimethylpropyl)amino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 90 minutes. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for 75 minutes under a pressure of 0.1 to 0.5 mm Hg. The flask is removed from the metal bath and is allowed to cool while the polymer solidifies. The resulting dark red polyester, containing 30.3 weight percent of the anthraquinone colorant residue, has an inherent viscosity of 0.56, no melting temperature, a weight average molecular weight of 39,000, a number average molecular weight of 20,000 and a polydispersity value of 1.94.

### EXAMPLE 17

A portion (25.0 g) of the amorphous polyester color concentrate prepared in PREPARATION 3 is granulated using a Wiley mill and dissolved in methylene chloride (200 mL) at about 25°C with stirring. Ethyl acetate (200 mL) is added and the methylene chloride is removed by distillation. The mixture is stirred for about 12 to 15 hours (usually overnight) at about 25°C during which time the colored semicrystalline powder separates. Acetone (200 mL) is added with stirring and the solid is collected by filtration and slurried in acetone (200 mL) and filtered four times to remove oligomers from the product which after drying weighs 23.6 g. The colored semicrystalline powder thus prepared has an inherent viscosity of 0.58, a melting temperature of 144°C, a weight average molecular weight of 38,000, a number average molecular weight of 25,000 and a polydispersity value of 1.52. The accountability of the anthraquinone colorant compound is 93% as determined by visual spectroscopy and a comparison of the absorbance of a methylene chloride solution of 1,5-bis[(3-hydroxy-2,2-dimethylpropyl)amino]anthraquinone with the absorbance of a methylene chloride solution of the microcrystalline color concentrate. The comparison shows no shift in absorbance indicating that the colorant is not decomposed during the synthesis of the polyester.

### EXAMPLE 18

The procedure of PREPARATION 3 is repeated using:
108.6 g (0.560 mol) dimethyl terephthalate
75.7 g (0.728 mol) 2,2-dimethyl-1,3-propanediol
0.0170 g Ti from a n-butanol solution of titanium tetraisopropoxide
52.0 g (0.44 mol) 1,5-bis[(3-hydroxy-2,2-dimethylpropyl)amino]anthraquinone
The dark red polyester color concentrate contains 30.6 weight percent of the residue of 1,5-bis[(3-hydroxy-2,2-dimethylpropyl)amino]anthraquinone and has an inherent viscosity of 0.47, a weight average molecular weight of 31,000, a number average molecular weight of 17,000 and a polydispersity value of 1.84.

The procedure described in Example 17 is repeated precisely using 25.0 g of the color concentrate to give 22.4 g of dark red, semicrystalline powder having an inherent viscosity of 0.49, a weight average molecular weight of 35,000, a number average molecular weight of 25,000 and a polydispersity value of 1.42. The accountability of the anthraquinone colorant by visual spectroscopy is 91.1%.

### EXAMPLE 19

A portion (57.2 g) of the color concentrate prepared in Example 18 is granulated and partially dissolved in boiling ethyl acetate (480 mL) by stirring. The mixture is cooled with stirring to 25°C at a rate of less than 1°C per minute. Stirring is stopped and the color concentrate is allowed to precipitate and undergo solvent-induced crystallization for about 15 hours. The dark red crystalline solid is separated by filtration and slurried in acetone (300 mL) three or four times to remove any low molecular weight oligomers from the product. After the last filtration, the solid is dried to give 45.1 g of colored semicrystalline powder having a melting temperature of 122°C, a weight average molecular weight of 36,122, a number average molecular weight of 26,224 and a polydispersity value of 1.38. Color accountability, determined as described in Example 17, is 93%.

### EXAMPLE 20

The procedure of PREPARATION 3 is repeated using:
84.1 g (0.43 mol) dimethyl terephthalate
58.6 g (0.56 mol) 2,2-dimethyl-1,3-propanediol
0.0165 g Ti from a n-butanol solution of titanium tetraisopropoxide
85.1 g (0.21 mol) 1,5-bis[(3-hydroxy-2,2-dimethylpropyl)amino]anthraquinone
The extremely dark red polyester color concentrate contains 51.6 weight percent of the residue of 1,5-bis[(3-hydroxy-2,2-dimethylpropyl)amino]anthraquinone and has an inherent viscosity of 0.36.

The procedure described in Example 17 is repeated precisely using 25.0 g of the color concentrate to give 22.9 g of very dark red, semicrystalline powder.

### EXAMPLE 21

A portion (75.0 g) of the color concentrate prepared in Example 20 is ground and crystallized from ethyl acetate (500 mL) as described in Example 19 to obtain 58.8 g of very dark red crystalline color concentrate with an anthraquinone colorant accountability of 93%.

### EXAMPLE 22

The following materials are placed in a 500-mL three-necked, round-bottom flask:
145.50 g (0.750 mol) dimethyl terephthalate
101.25 g (1.125 mol) 1,4-butanediol
0.0214 g Ti from a n-butanol solution of titanium tetraisopropoxide
63.00 g (0.044 mol) 1,5-bis[(3-hydroxy-2,2-dimethylpropyl)amino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C. The mixture is heated at 200°C for 1.75 hours and then the temperature is raised to and maintained at 225°C over a period of 2.25 hours. The nitrogen sweep is then stopped and vacuum is applied to lower the pressure to about 0.5 to 1.0 mm Hg. The polycondensation is completed by heating the flask and contents at about 225°C for 1 hour under a pressure of 0.5 to 1.0 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Hexane (700 mL) is added slowly with stirring to dilute the slurry and keep it stirrable. (Acetone, which removes more oligomeric material, also may be used.) The diluted slurry is stirred for 30 minutes, filtered and the cake is washed with hexane. The cake is twice reslurried in hexane and then dried in a vacuum oven. The resulting dark red semicrystalline polyester powder, containing 29.42 weight percent of the anthraquinone colorant residue, has an inherent viscosity of 0.202, a melting temperature of 175°C, a glass transition temperature of 66°C, a weight average molecular weight of 12,646, a number average molecular weight of 8,359 and a polydispersity value of 1.51. Reslurrying the powder twice in acetone increases the non-extractability of color and raises the inherent viscosity to 0.26.

### EXAMPLE 23

The procedure described in Example 22 is repeated except that the reaction mixture is heated at 200°C for 1.75 hours and then the temperature is raised to 220°C over 1.25 hours, then to 240°C over 1.25 hours and finally to 270°C over 1.25 hours. Vacuum is applied to lower the pressure to about 0.5 to 1.0 mm Hg and polycondensation is completed by heating the flask and contents at about 270°C for 22 minutes under a pressure of 0.5 to 1.0 mm Hg. The vacuum is then relieved with nitrogen and methyl benzoate (125 mL) is added slowly and stirred to solution over about 50 minutes with the flask still in the metal bath. The resulting solution is transferred to a 2 L beaker and stirred until crystallization occurs. Acetone (1 L) is added slowly with stirring to dilute the slurry and keep it stirrable. The diluted slurry is stirred for 30 minutes, filtered and the cake is slurried in hexane (1 L). The cake is again slurried in acetone and hexane and then dried in air to yield 212.89 g (99.40% of theory) colored powder. The dark red polyester powder, containing 29.42 weight percent of the anthraquinone colorant residue, has an inherent viscosity of 0.190, a melting temperature of 172°C, a glass transition temperature of 54°C, a weight average molecular weight of 12,806, a number average molecular weight of 8,903 and a polydispersity value of 1.44.

### EXAMPLE 24

The following materials are placed in a 500-mL, three-necked, round-bottom flask:
91.0 g (0.469 mol) dimethyl terephthalate
63.3 g (0.704 mol) 1,4-butanediol
0.01343 g Ti from a n-butanol solution of titanium tetraisopropoxide
40.0 g (0.0976 mol) 1,5-bis[(3-hydroxy-2,2-dimethylpropyl)amino]anthraquinone
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for 45 minutes under a pressure of 0.1 to 0.5 mm Hg. The vacuum is then relieved with nitrogen and methyl benzoate (125 mL) is added slowly and stirred to solution over about 10 minutes with the flask still in the metal bath. The resulting solution is transferred to a 2 L beaker and stirred until crystallization occurs. Acetone (500 mL) is added slowly with stirring to dilute the slurry and keep it stirrable. The diluted slurry is stirred for 30 minutes, filtered and the cake is washed with acetone. The cake is twice reslurried in acetone and then dried in air. The resulting dark red semicrystalline polyester powder, containing 29.77 weight percent of the anthraquinone colorant residue, has an inherent viscosity of 0.485, a melting temperature of 182°C, a weight average molecular weight of 36,927, a number average molecular weight of 23,685 and a polydispersity value of 1.59.

### PREPARATION 4

The following materials are placed in a 500-mL three-necked, round-bottom flask:
125.1 g (0.645 mol) dimethyl terephthalate
94.6 g (0.91 mol) 2,2-dimethyl-1,3-propanediol
0.01864 g Ti from a n-butanol solution of titanium tetraisopropoxide
36.3 g (0.11 mol) methine colorant having the structure: 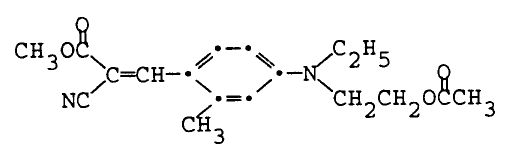
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 90 minutes. Over the next 30 minutes the temperature is increased to about 240°C and then to about 260°C over the next 30 minutes. The temperature is quickly raised (over about 10 minutes) to 275°C with a stream of nitrogen bleeding into the system and a vacuum is applied until the pressure is reduced to 0.5 mm Hg. The polycondensation is completed by heating the flask and contents at about 275°C for about 1.25 hours under a pressure of 0.1 to 0.5 mm Hg. The flask is removed from the metal bath and is allowed to cool while the polymer solidifies. The resulting high molecular weight yellow polyester, containing 19.76 weight percent of the methine colorant residue, has an inherent viscosity of 0.30, no melting temperature, a weight average molecular weight of 21,691, a number average molecular weight of 13,366 and a polydispersity value of 1.63.

### EXAMPLE 25

A portion (25.0 g) of the amorphous polyester color concentrate prepared in PREPARATION 4 is granulated using a Wiley mill and dissolved in methylene chloride (200 mL) at about 25°C with stirring. Ethyl acetate (200 mL) is added and the methylene chloride is removed by distillation. The mixture is stirred for about 12 to 15 hours (usually overnight) at about 25°C during which time the colored semicrystalline powder separates. The solid is collected by filtration and reslurried in acetone (200 mL each time) and filtered four times to remove oligomers from the product which after drying weighs 20.0 g. The colored semicrystalline powder thus prepared has an inherent viscosity of 0.35, a melting temperature of 134°C, a weight average molecular weight of 23,793, a number average molecular weight of 17,323 and a polydispersity value of 1.37. The total accountability of the methine colorant compound is 93% as determined by visual spectroscopy and a comparison of the absorbance of a methylene chloride solution of the starting methine colorant reactant with the absorbance of a methylene chloride solution of the color concentrate. The comparison shows no shift in absorbance indicating that the colorant is not decomposed during the synthesis of the polyester.

### EXAMPLE 26

The following materials are placed in a 500-mL, three-necked, round-bottom flask:
97.0 g (0.50 mol) dimethyl terephthalate
62.0 g (1.00 mol) ethylene glycol
0.0120 g Ti from a n-butanol solution of titanium tetraisopropoxide
36.0 g (0.109 mol) methine colorant used in PREPARATION 4
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 75 minutes. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for about 45 to 60 minutes under a pressure of 0.1 to 0.5 mm Hg. Upon completion of polycondensation, the vacuum is relieved with nitrogen and methyl benzoate (160 mL) is added slowly. The mixture is stirred to solution over about 10 minutes with the flask still in the metal bath. The heat is then removed and stirring continued. Crystallization begins to occur at about 115°C. At 50°C, acetone (150 mL) is added to facilitate stirring. The diluted slurry is stirred for about 30 minutes, filtered and the cake washed three times with acetone and dried. The resulting dark yellow polyester contains 29.9 weight percent of the methine colorant residue, has an inherent viscosity of 0.29, a weight average molecular weight of 30,518, a number average molecular weight of 16,889 and a polydispersity value of 1.80. The weight of polyester color concentrate powder recovered is 104.7 g, 86.9% of theory.

### EXAMPLE 27

The procedure described in Example 26 is repeated except that upon completion of the polycondensation, the vacuum is relieved with nitrogen and diphenyl oxide (200 mL) is added dropwise and the mixture is stirred to solution. Heating is discontinued and crystallization begins to occur at about 130°C. At about 100°C, acetone (100 mL) is added to facilitate stirring. The diluted slurry is filtered and the cake washed well with acetone and dried in air (yield - 100.5 g). The resulting dark yellow polyester contains 29.9 weight percent of the methine colorant residue, has an inherent viscosity of 0.67, a melting temperature of 176°C, a glass transition temperature of 80°C, a weight average molecular weight of 46,040, a number average molecular weight of 22,502 and a polydispersity value of 2.05.

### EXAMPLE 28

The following materials are placed in a 500-mL, three-necked, round-bottom flask:
97.0 g (0.50 mol) dimethyl terephthalate
67.5 g (0.75 mol) 1,4-butanediol
0.0142 g Ti from a n-butanol solution of titanium tetraisopropoxide
45.0 g (0.136 mol) methine colorant used in PREPARATION 4
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 30 minutes the temperature is increased to about 240°C and then to about 260°C over the next 30 minutes. The temperature is quickly raised (over about 10 minutes) to 275°C and a vacuum is applied until the pressure is reduced to 0.5 mm Hg. The polycondensation is completed by heating the flask and contents at about 275°C for about 45 minutes under a pressure of 0.1 to 0.5 mm Hg. Upon completion of the polycondensation, the vacuum is relieved with nitrogen and methyl benzoate (125 mL) is added slowly and the mixture is stirred to solution with the flask still in the metal bath. The resulting solution is transferred to a 2 L beaker and stirred until crystallization occurs. Acetone (500 mL) is added slowly with stirring to dilute the slurry and keep it stirrable. The diluted slurry is stirred for 30 minutes filtered and the cake washed with acetone. The cake is twice reslurried in acetone and then dried in air. The resulting dark yellow semicrystalline polyester powder contains 31.5 weight percent of the methine colorant residue, has an inherent viscosity of 0.359, a melting temperature of 185°C, a weight average molecular weight of 29,385, a number average molecular weight of 17,655 and a polydispersity value of 1.66. The weight of the powder recovered is 129.4 g, 90.8% of theory.

### EXAMPLE 29

The following materials are placed in a 500-mL three-necked, round-bottom flask:
155.2 g (0.80 mol) dimethyl terephthalate
108.0 g (1.20 mol) 1,4-butanediol
0.0226 g Ti from a n-butanol solution of titanium tetraisopropoxide
70.0 g (0.212 mol) methine colorant used in PREPARATION 4
The flask is equipped with a nitrogen inlet, stirrer, vacuum outlet, and condensing flask. The flask and contents are heated in a Belmont metal bath with a nitrogen sweep over the reaction mixture as the temperature is increased to 200°C and then to 220°C over 2 hours. Over the next 90 minutes the temperature is increased to about 230°C and a vacuum is applied until the pressure is reduced to 0.5 mm Hg. The polycondensation is completed by heating the flask and contents at about 230°C for about 8 hours under a pressure of 0.1 to 0.5 mm Hg. The vacuum is relieved with nitrogen and methyl benzoate (200 mL) is added slowly and the mixture is stirred to solution over about 10 minutes with the flask still in the metal bath. The resulting solution is transferred to a 2 L beaker and stirred until crystallization occurs. Hexane (800 mL) is added slowly with stirring to dilute the slurry and keep it stirrable. The diluted slurry is stirred for 30 minutes, filtered and the cake is washed with acetone. The cake is twice reslurried in acetone and the dried in air. The resulting dark yellow semicrystalline polyester powder contains 30.87 weight percent of the methine colorant residue, has an inherent viscosity of 0.550, a melting temperature of 179°C, a weight average molecular weight of 33,707, a number average molecular weight of 19,956 and a polydispersity value of 1.69. The weight of the powder recovered is 213.4 g.

### EXAMPLE 30

The procedure described in Reference Example 4 is repeated using the following materials:
126.5 g (0.652 mol) dimethyl terephthalate
94.6 g (0.91 mol) 2,2-dimethyl-1,3-propanediol
0.01864 g Ti from a n-butanol solution of titanium tetraisopropoxide
37.0 g (0.096 mol) methine colorant having the structure: 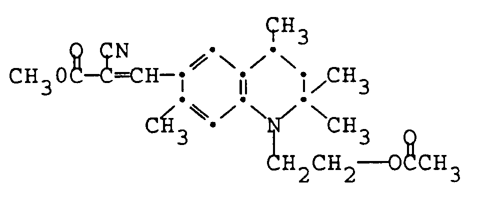
The resulting dark yellow polyester contains 19.8 weight percent of the methine colorant residue, has an inherent viscosity of 0.38, no melting temperature, a weight average molecular weight of 27,625, a number average molecular weight of 13,770 and a polydispersity value of 1.78. A portion (25.0 g) of the amorphous color concentrate is crystallized according to the procedure of Example 25 to obtain an essentially theoretical yield of colored semi-crystalline powder having an inherent viscosity of 0.37, a weight average molecular weight of 27,625, a number average molecular weight of 19,130, a polydispersity value of 1.4 and a melting temperature of about 130°C.

### EXAMPLE 31

The following materials are charged to the reactor:
11.90 lbs. (27.8 mol) dimethyl terephthalate
12.47 lbs. (62.8 mol) 1,4-butanediol
14.58 g Ti from a n-butanol solution of titanium tetraisopropoxide
1.50 lbs. (1.42 mol) 1,5-bis(carboxyanilino)anthraquinone
The jacketed reactor is equipped with a nitrogen inlet, stirrer, vacuum outlet, and condenser, and is heated by oil circulating through the jacket. The reactor is continuously purged with nitrogen while the contents of the reactor are heated to 180°C over two hours. The temperature is held for 30 minutes at 180°C, then increased to 200°C over 30 minutes. The temperature is held for 30 minutes at 200°C, then increased to 220°C over 20 minutes. Over the next 90 minutes the temperature is increased to 230°C and a vacuum is applied until the pressure is reduced to 3900 microns Hg. The polycondensation is completed by holding the reactor contents at 230°C and about 2500 microns Hg pressure for 15 minutes. The reactor is then pressurized with nitrogen to extrude the contents of the reactor into water. The extruded polymer is granulated by grinding in a Wiley mill, then dried under vacuum at 80°C for four hours.

The resulting dark red polyester contains 10.4 weight percent of the anthraquinone colorant residue and has an inherent viscosity of 0.24. With the gel permeation chromatography detector at 254 nm, the polyester has a weight average molecular weight of 13,182, a number average molecular weight of 8,856, and a polydispersity value of 1.49. With the detector at 546 nm, the weight average molecular weight is 12,855, the number average molecular weight is 8,210, and the polydispersity value is 1.57. The yield of the polyester is 85.4%.

A portion (700 g) of the partially-crystalline color concentrate is added to 3500 ml ethylene glycol diacetate in a 5 L round-bottom flask. The mixture is heated to the boiling point of the solvent (188°C) and refluxed for one hour, which dissolves the color concentrate. The solution is cooled to room temperature to precipitate the solid semicrystalline material. The solid product is collected by filtration, washed on the filter with 1400 ml ethylene glycol diacetate, and dried under vacuum at 100°C for 48 hours. The resulting dark red semicrystalline polyester powder contains 9.4 weight percent of the anthraquinone colorant residue, has an inherent viscosity of 0.24, and a melting temperature of 210°C. With the gel permeation chromatography detector at 254 nm, the powder has a weight average molecular weight of 14,080, a number average molecular weight of 10,122, and a polydispersity value of 1.39. With the detector set at 546 nm, the weight average molecular weight is 14,252, the number average molecular weight is 10,823, and the polydispersity value is 1.32. The weight of the powder recovered from the dissolution-crystallization-precipitation process is 675.6 g (96.5% yield). After dispersing a portion of the powder in castor oil by milling on a Hoover muller, the colorant does not bleed into the castor oil. The powder is ground by a Trost TX air impact pulverizer to give an average particle size of 3.9 microns; as measured by the Microtrac particle size analyzer.

### PREPARATION 5

Example 4 above is repeated except the blue colored polymer is not crystallized from methyl benzoate. After the polycondensation step is completed at 275°C, the flask is removed from the metal bath and allowed to cool while the polymer solidifies. The color concentrate is granulated by grinding to a particle size of about 3 mm using a Wiley mill.

### PREPARATION 6

Example 22 above is repeated-except the red colored polymer is not crystallized from methyl benzoate. After the polycondensation step is completed at 275°C, the flask is removed from the metal bath and allowed, to cool while the polymer solidifies. The color concentrate is granulated by grinding to a particle size of about 3 mm using a Wiley mill.

### PREPARATION 7

Example 29 above is repeated except the yellow colored polymer is not crystallized from methyl benzoate. After the polycondensation reaction is completed at 275°C., the flask is removed from the metal bath and allowed to cool while the polymer solidifies. The color concentrate is granulated by grinding to a particle size of about 3 mm using a Wiley mill.

### EXAMPLE 32

The yellow polymeric colorant of Example 29 (2.0 g), the red polymeric colorant of Example 22 (3.5 g) and the blue polymeric colorant of Example 4 (4.5 g) are added to a flask which contains methyl benzoate (100 ml). Heating and stirring under nitrogen are continued for about 1 hour with the temperature rising to about 200°C. The heat is removed and the solution allowed to cool to room temperature with stirring whereupon the black colored polyester is crystallized. Hexane (100 ml) is added slowly with stirring. The diluted slurry is stirred for 15 min, filtered and the cake washed twice with hexane and air dried. A reddish-black colored semicrystalline powder (9.54 g) is obtained which has an inherent viscosity of 0.158, a melting temperature of 177°C, a weight average molecular weight of 8,714 and a number average molecular weight of 2,636.

### EXAMPLE 33

Example 32 is repeated using the blue polymer of PREPARATION 5 (4.5 g) the red polymer of PREPARATION 6 (3.5 g) and the yellow polymer of PREPARATION 7 (2.0 g). A reddish-black semicrystalline powder is obtained.

### EXAMPLE 34

The following materials are placed in a 500-ml three-necked, round-bottom flask:
111.6 g (0.575 mol) dimethyl terephthalate
81.0 g (0.90 mol) 1,4-butanediol
0.0141 g Ti from n-butanol solution of titanium tetraisopropoxide
4.73 g (.00989 mol) red colorant [1,5-bis(o-carboxyanilino)anthraquinone]
4.73 g (.00924 mol) yellow colorant [1,5-bis(o-carboxyphenylthio)anthraquinone]
4.73 g (.00588 mol) blue colorant having the structure 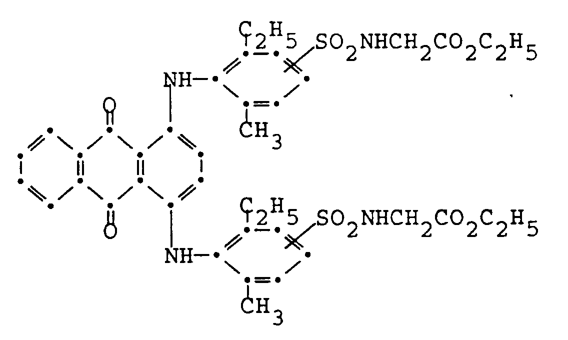
The flask is equipped with a nitrogen inlet, stirrer, vacuum outlet and condensing flask. The flask and contents are heated in a Belmont metal bath with a nitrogen sweep over the reaction mixture as the temperature is increased to 200°C and then to 225°C over two hours. Over the next hour, the temperature is increased to about 240°C and then to 250°C over the next 15 minutes and a vacuum is applied until the pressure is reduced to about 0.5 mm Hg. The polycondensation is completed by heating the flask and contents at about 250°C for 1 hour. Upon completion of the polycondensation, the vacuum is relieved and methyl benzoate (125 ml) is added slowly and the mixture is stirred to solution with the flask still in the metal bath. The resulting solution is transferred to a 2 L beaker and stirred until crystallization occurs. Acetone (500 ml) is added slowly with stirring to dilute the slurry and keep it stirrable. The diluted slurry is stirred for 30 minutes, filtered and the cake washed with acetone. The cake is twice reslurried in acetone and then dried in air to give a reddish-black powder.

### EXAMPLE 35

Example 23 is repeated except finely divided titanium dioxide (TiO₂) is added (2.14 g) along with the reactants. The bright red polyester powder, containing 29.42 weight percent of the anthraquinone colorant and 1% of TiO₂, has an inherent viscosity of 0.146, a melting temperature of 182°C, a glass transition temperature of 37°C, a weight average molecular weight of 8,218, a number average molecular weight 6,235 and a polydispersity value of 1.32. The weight of powder recovered is 198.6 g, 91.8% of the theoretical weight.

## Claims

1. A semicrystalline powder colorant composition having an average particle size of less than 50 microns comprising a polyester which has been modified by dissolution-crystallization-precipitation to impart crystallinity thereto having copolymerized therein at least 1.0 weight percent, based on the weight of the composition, of the residue of one or more difunctional colorant compounds which are stable under polyester polymerization conditions, provided that the colorant compound is not a methine compound.

2. A semicrystalline powder colorant composition according to Claim 1 comprising a normally-amorphous polyester having an inherent viscosity of at least 0.2 which has been modified by dissolution-crystallization-precipitation to impart crystallinity thereto comprised of:
(i) diacid residues comprised of at least 50 mole percent terephthalic and/or 2,6-naphthalenedicarboxylic acid residues;
(ii) diol residues comprised of at least 50 mole percent of the residue of a diol having the formula: 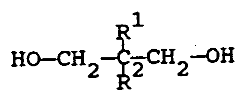 and
wherein R¹ is hydrogen or C₁-C₄ alkyl and R² is C₁-C₄ alkyl; and
(iii) at least 5.0 weight percent, based on the weight of the composition, residues of one or more difunctional colorant compounds which are stable under polyester polymerization conditions, provided that the colorant compound is not a methine compound.

3. A semicrystalline powder colorant composition according to Claim 1 having an average particle size of less than 50 microns comprising a normally-amorphous polyester having an inherent viscosity of about 0.2 to 0.8 which has been modified by dissolution-crystallization-precipitation to impart crystallinity thereto comprised of:
(i) diacid residues comprised of at least 80 mole percent terephthalic and/or 2,6-naphthalenedicarboxylic acid residues;
(ii) diol residues comprised of at least 80 mole percent of the residue of a diol having the formula: 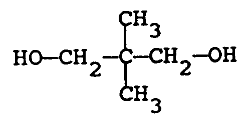 and
(iii) at least 5.0 weight percent, based on the weight of the composition, residues of a difunctional colorant compound which are stable under polyester polymerization conditions, provided that the colorant compound is not a methine compound.

4. A semicrystalline powder colorant composition according to Claim 2 wherein component (iii) constitutes about 10 to 40 weight percent of the composition.

5. A semicrystalline powder colorant composition according to Claim 1 having an average particle size of less than 50 microns comprising a partially-crystalline polyester which had been modified by dissolution-crystallization-precipitation to impart increased crystallinity thereto comprised of:
(i) diacid residues comprised of at least 80 mole percent terephthalic acid residues, 2,6-naphthalenedicarboxylic acid residues, 1,3-cyclohexanedicarboxylic acid residues, 1,4-cyclohexanedicarboxylic acid residues or a mixture thereof;
(ii) diol residues comprised of at least 50 mole percent of residues having the formula -O-(CH₂)ₚ-O- wherein p is 2 to 12; and
(iii) at least 5.0 weight percent, based on the weight of the composition, residues of one or more difunctional colorant compounds which are stable under polyester polymerization conditions, provided that the colorant compound is not a methine compound.

6. A semicrystalline powder colorant composition according to Claim 5 comprised of:
(i) diacid residues comprised of at least 80 mole percent terephthalic acid residues, 2,6-naphthalenedicarboxylic acid residues, 1,3-cyclohexanedicarboxylic acid residues, 1,4-cyclohexanedicarboxylic acid residues or a mixture thereof;
(ii) diol residues comprised of at least 50 mole percent of residues having the formula -O-(CH₂)ₚ-O- wherein p is 4 to 12; and
(iii) at least 5.0 weight percent, based on the weight of the composition, residues of one or more difunctional colorant compounds which are stable under polyester polymerization conditions, provided that the colorant compound is not a methine compound.

7. A semicrystalline powder colorant composition according to Claim 5 comprising a polyester having an inherent viscosity of at least 0.20 and a melting temperature of at least 110°C comprised of:
(i) diacid residues comprised of at least 80 mole percent terephthalic acid residues;
(ii) diol residues comprised of at least 80 mole percent of residues of 1,4-butanediol; and
(iii) about 10 to 40 weight percent, based on the weight of the composition, residues of one or more difunctional colorant compounds which are stable under polyester polymerization conditions, provided that the colorant compound is not a methine compound.

8. A semicrystalline powder colorant composition according to Claim 6 wherein component (iii) constitutes about 10 to 40 weight percent of the composition.

9. A process for the preparation of a semicrystalline powder colorant composition of Claim 1 which comprises:
(1) dissolving in an inert, organic solvent a polyester color concentrate comprising a polyester having copolymerized therein at least 1.0 weight percent of the residues of at least one difunctional colorant compound which are stable under polyester polymerization conditions; provided that the colorant compound is not a methine compound, and
(2) precipitating from the solution of step (1) the semicrystalline colorant composition in a finely divided form comprised of particles having an average particle size of less than 50 microns.

10. A process according to Claim 9 wherein dissolution is performed at a temperature of about 25°C up to the boiling point of the inert solvent and the inert solvent is selected from aliphatic chlorides, alkyl carboxylic acid esters having 3 to about 10 carbon atoms and mixtures thereof.

11. A process for the preparation of a semicrystalline powder colorant composition of Claim 1 which comprises:
(1) dissolving in an inert, organic solvent selected from aliphatic chlorides, alkyl carboxylic acid esters having 3 to about 10 carbon atoms and mixtures thereof an amorphous polyester color concentrate having an inherent viscosity of at least 0.2 comprising:
(i) diacid residues comprised of at least 50 mole percent terephthalic and/or 2,6-naphthalenedicarboxylic acid residues;
(ii) diol residues comprised of at least 50 mole percent of the residue of a diol having the formula: 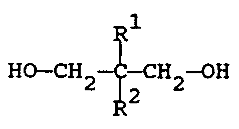 wherein R¹ is hydrogen or C₁-C₄ alkyl and R² is C₁-C₄ alkyl; and
(iii) at least 5.0 weight percent, based on the weight of the composition, of residues of one or more difunctional colorant compounds which are stable under polyester polymerization conditions copolymerized in the polyester; provided that the colorant compound is not a methine compound, and
(2) precipitating from the solution of step (1) the semicrystalline colorant composition in a finely divided form consisting of particles having an average particle size of less than 50 microns.

12. A process according to Claim 11 wherein the amorphous polyester has an inherent viscosity of about 0.2 to 0.8 and is comprised of:
(i) diacid residues comprised of at least 80 mole percent terephthalic and/or 2,6-naphthalene-dicarboxylic acid residues;
(ii) diol residues comprised of at least 80 mole percent of the residue of a diol having the formula: 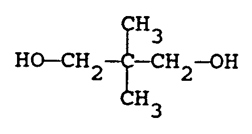 and
(iii) about 10 to 40 weight percent, based on the weight of the composition, of residues of a difunctional colorant compound which are stable under polyester polymerization conditions copolymerized in the polyester, provided that the colorant compound is not a methine compound.

13. A process according to Claim 11 wherein step (1) comprises dissolving a melt of the partially crystalline polyester in an inert solvent selected from C₁-C₄ alkyl esters of aromatic mono- and dicarboxylic acids.

14. A process for the preparation of a semicrystalline powder colorant composition of claim 1 which comprises:
(1) dissolving in an inert, organic solvent selected from aliphatic chlorides, alkyl carboxylic acid esters having 3 to about 10 carbon atoms and mixtures thereof a partially crystalline polyester color concentrate having an inherent viscosity of at least 0.2 comprising:
(i) diacid residues comprised of at least 80 mole percent terephthalic acid residues, 2,6-naphthalenedicarboxylic acid residues, 1,3-cyclohexanedicarboxylic acid residues, 1,4-cyclohexanedicarboxylic acid residues or a mixture thereof;
(ii) diol residues comprised of at least 50 mole percent of residues having the formula -O-(CH₂)ₚ-O- wherein p is 2 to 12; and
(iii) at least 5.0 weight percent, based on the weight of the composition, of residues of one or more difunctional colorant compounds which are stable under polyester polymerization conditions, provided that the colorant compound is not a methine compound.
(2) precipitating from the solution of step (1) the semicrystalline colorant composition in a finely divided form comprised of particles having an average particle size of less than 50 microns.

15. A process according to Claim 14 wherein the partially crystalline polyester comprises:
(i) diacid residues comprised of at least 80 mole percent terephthalic acid residues, 2,6-naphthalenedicarboxylic acid residues, 1,3-cyclohexanedicarboxylic acid residues, 1,4-cyclohexanedicarboxylic acid residues or a mixture thereof;
(ii) diol residues comprised of at least 50 mole percent of residues having the formula -O-(CH₂)ₚ-O- wherein p is 4 to 12; and
(iii) at least 5.0 weight percent, based on the weight of the composition, of residues of one or more difunctional colorant compounds which are stable under polyester polymerization conditions copolymerized in the polyester, provided that the colorant compound is not a methine compound.

16. A process according to Claim 14 wherein the partially crystalline polyester has an inherent viscosity of at least 0.2 and a melting temperature of at least 110°C and comprises:
(i) diacid residues comprised of at least 80 mole percent terephthalic acid residues;
(ii) diol residues comprised of at least 80 mole percent of residues of 1,4-butanediol; and
(iii) about 10 to 40 weight percent, based on the weight of the composition, of residues of one or more difunctional colorant compounds which are stable under polyester polymerization conditions copolymerized in the polyester, provided that the colorant compound is not a methine compound.

17. A process according to Claim 14 wherein step (1) comprises dissolving a melt of the partially crystalline polyester in an inert solvent selected from C₁-C₄ lower alkyl esters of aromatic mono- and di-carboxylic acids, or glycol esters of C₁-C₄ aliphatic carboxylic acids.

## Patentansprüche

1. Eine semikristalline Pulver-Färbezusammensetzung mit einer durchschnittlichen Teilchengröße von weniger als 50 Mikrometer, enthaltend einen Polyester, der durch Auflösung - Kristallisierung - Ausfällung modifiziert wurde, um diesem Kristallinität zu verleihen, wobei dieser mit mindestens 1,0 Gewichtsprozent, bezogen auf das Gewicht der Zusammensetzung, einer Gruppe einer oder mehrerer difunktionaler Farbstoffverbindungen copolymerisiert ist, welche unter Polyester-Polymerisierungsbedingungen stabil sind, vorausgesetzt daß die Farbstoffverbindung nicht eine Methinverbindung ist.

2. Semikristalline Pulver-Farbstoffzusammensetzung nach Anspruch 1, enthaltend einen normalerweise amorphen Polyester mit einer inhärenten Viskosität von mindestens 0,2, der durch Auflösung - Kristallisierung - Ausfällung modifiziert wurde, um ihm Kristallinität zu verleihen, und welcher besteht aus:
(i) Disäuregruppen, bestehend aus mindestens 50 mol-% Terephthal- und/oder 2,6-Naphthalindicarbonsäuregruppen;
(ii) Diolgruppen, bestehend aus mindestens 50 mol-% einer Diolgruppe mit der Formel: 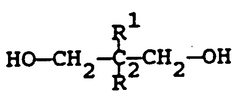 und
worin R¹ Wasserstoff oder C₁-C₄-Alkyl und R² C₁-C₄-Alkyl ist und
(iii) mindestens 5,0 Gewichtsprozent, bezogen auf das Gewicht der Zusammensetzung, Gruppen einer oder mehrerer difunktionaler Farbstoffverbindungen, die unter Polyester-Polymerisierungsbedingungen stabil sind, vorausgesetzt daß die Farbstoffverbindung nicht eine Methinverbindung ist.

3. Semikristalline Pulver-Färbezusammensetzung gemäß Anspruch 1, mit einer durchschnittlichen Teilchengröße von weniger als 50 Mikrometer, enthaltend einen normalerweise amorphen Polyester mit einer inhärenten Viskosität von etwa 0,2 bis 0,8, der durch Auflösung - Kristallisierung - Ausfällung modifiziert wurde, um ihm Kristallinität zu verleihen, und welcher besteht aus:
(i) Disäuregruppen, bestehend aus mindestens 80 mol-% Terephthal- und/oder 2,6-Naphthalindicarbonsäuregruppen;
(ii) Diolgruppen, bestehend aus mindestens 80 mol-% einer Diolgruppe mit der Formel: 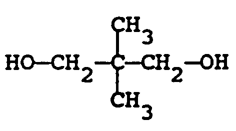 und
(iii) mindestens 5 Gewichtsprozent, bezogen auf das Gewicht der Zusammensetzung, Gruppen einer difunktionalen Farbstoffverbindung, die unter Polyester-Polymerisierungsbedingungen stabil sind, vorausgesetzt daß die Farbstoffverbindung nicht eine Methinverbindung ist.

4. Semikristalline Pulver-Färbezusammensetzung nach Anspruch 2, wobei der Bestandteil (iii) 10 bis 40 Gewichtsprozent der Zusammensetzung ausmacht.

5. Semikristalline Pulver-Färbezusammensetzung nach Anspruch 1, mit einer durchschnittlichen Teilchengröße von weniger als 50 Mikrometer, bestehend aus einem teilweise kristallinen Polyester, der durch Auflösung - Kristallisierung - Ausfällung modifiziert wurde, um ihm eine erhöhte Kristallinität zu verleihen, und welcher besteht aus:
(i) Disäuregruppen, bestehend aus mindestens 80 mol-% Terephthalsäuregruppen, 2,6-Naphthalindicarbonsäuregruppen, 1,3-Cyclohexandicarbonsäuregruppen, 1,4-Cyclohexandicarbonsäuregruppen oder Mischungen davon;
(ii) Diolgruppen, bestehend aus mindestens 50 mol-% Gruppen mit der Formel -O-(CH₂)ₚ-O- wobei p 2 bis 12 ist und
(iii) mindestens 5 Gewichtsprozent, bezogen auf das Gewicht der Zusammensetzung, Gruppen einer oder mehrerer difunktionaler Farbstoffverbindungen, die unter Polyester-Polymerisierungsbedingungen stabil sind, vorausgesetzt daß die Farbstoffverbindung nicht eine Methinverbindung ist.

6. Semikristalline Pulver-Färbezusammensetzung nach Anspruch 5, bestehend aus:
(i) Disäuregruppen, bestehend aus mindestens 80 mol-% Terephthalsäuregruppen, 2,6-Naphthalindicarbonsäuregruppen, 1,3-Cyclohexandicarbonsäuregruppen, 1,4-Cyclohexandicarbonsäuregruppen oder Mischungen davon;
(ii) Diolgruppen, bestehend aus mindestens 50 mol-% Gruppen mit der Formel -O-(CH₂)ₚ-O- wobei p 4 bis 12 ist und
(iii) mindestens 5 Gewichtsprozent, bezogen auf das Gewicht der Zusammensetzung, Gruppen einer oder mehrerer difunktionaler Farbstoffverbindungen, die unter Polyester-Polymerisierungsbedingungen stabil sind, vorausgesetzt daß die Farbstoffverbindung nicht eine Methinverbindung ist.

7. Semikristalline Pulver-Färbezusammensetzung nach Anspruch 5, bestehend aus einem Polyester mit einer inhärenten Viskosität von mindestens 0,20 und einer Schmelztemperatur von mindestens 110°C, aus:
(i) Disäuregruppen, bestehend aus mindestens 80 mol-% Terephthalsäuregruppen;
(ii) Diolgruppen, bestehend aus mindestens 80 mol-% 1,4-Butandiolgruppen;
(iii) etwa 10 bis 40 Gewichtsprozent, bezogen auf das Gewicht der Zusammensetzung, Gruppen einer oder mehrerer difunktionaler Farbstoffverbindungen, die unter Polyester-Polymerisierungsbedingungen stabil sind, vorausgesetzt daß die Farbstoffverbindung nicht eine Methinverbindung ist.

8. Semikristalline Pulver-Färbezusammensetzung nach Anspruch 6, wobei der Bestandteil (iii) etwa 10 bis 40 Gewichtsprozent der Zusammensetzung ausmacht.

9. Verfahren zur Herstellung einer semikristallinen PulverFärbezusammensetzung nach Anspruch 1, welches umfaßt:
(1) Auflösung eines Polyesterfarbstoffkonzentrats, das einen Polyester und darin copolymerisiert mindestens 1 Gewichtsprozent der Gruppen mindestens einer difunktionalen Farbstoffkomponente, die unter Polyester-Polymerisierungsbedingungen stabil ist, vorausgesetzt daß die Farbstoffverbindung nicht eine Methinverbindung ist, enthält, in einem inerten organischen Lösungsmittel;
(2) Ausfällung der semikristallinen Farbstoffzusammensetzung aus der Lösung des Schritts (1) in feinverteilter Form, bestehend aus Teilchen mit einer durchschnittlichen Teilchengröße von weniger als 50 Mikrometer.

10. Verfahren nach Anspruch 9, wobei die Auflösung bei einer Temperatur von etwa 25°C bis zum Siedepunkt des inerten Lösungsmittels durchgeführt wird und das inerte Lösungsmittel ausgewählt ist aus aliphatischen Chloriden, Alkylcarbonsäureestern mit 3 bis etwa 10 Kohlenstoffatomen und Mischungen davon.

11. Verfahren zur Herstellung einer semikristallinen Pulver-Färbezusammensetzung nach Anspruch 1, welches umfaßt:
(1) Auflösung in einem inerten organischen Lösungsmittel, ausgewählt aus aliphatischen Chloriden, Alkylcarbonsäureestern mit 3 bis etwa 10 Kohlenstoffatomen und Mischungen davon, eines amorphen Polyesterfarbstoffkonzentrats mit einer inhärenten Viskosität von mindestens 0,2 und enthaltend:
(i) Disäuregruppen, bestehend aus mindestens 50 mol-% Terephtal- und/oder 2,6-Naphthalindicarbonsäuregruppen;
(ii) Diolgruppen, bestehend aus mindestens 50 mol-% Gruppen eines Diols der Formel: 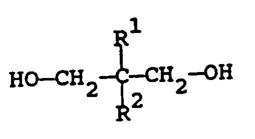 worin R¹ gleich Wasserstoff oder C₁-C₄-Alkyl und R² C₁-C₄-Alkyl ist und
(iii) mindestens 5,0 Gewichtsprozent, bezogen auf das Gewicht der Zusammensetzung, Gruppen eines oder mehrerer difunktionaler Farbstoffverbindungen, die unter Polyester-Polymerisierungsbedingungen stabil und in den Polyester copolymerisiert sind, vorausgesetzt daß die Farbstoffverbindung nicht eine Methinverbindung ist, und
(2) Ausfällung aus der Lösung des Schritts (1) der semikristallinen Färbezusammensetzung in feinverteilter Form bestehend aus Teilchen mit einer durchschnittlichen Teilchengröße von weniger als 50 Mikrometer.

12. Verfahren nach Anspruch 11, wobei der amorphe Polyester eine inhärente Vikosität von etwa 0,2 bis 0,8 hat und besteht aus:
(i) Disäuregruppen, bestehend aus mindestens 80 mol-% Terephthal- und/oder 2,6-Naphthalindicarbonsäuregruppen,
(ii) Diolgruppen, bestehend aus mindestens 80 mol-% der Diolgruppe mit der Formel: 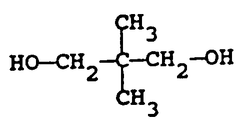 und
(iii) etwa 10 bis 40 Gewichtsprozent, bezogen auf das Gewicht der Zusammensetzung, Gruppen einer difunktionalen Farbstoffverbindung, die unter Polyester-Polymerisierungsbedingungen stabil und in den Polyester copolymerisiert sind, vorausgesetzt daß die Farbstoffverbindung nicht eine Methinverbindung ist.

13. Verfahren nach Anspruch 11, wobei Schritt (1) die Auflösung einer Schmelze des teilweise kristallinen Polyesters in einem inerten Lösungsmittel umfaßt, ausgewählt aus C₁-C₄-Alkylestern aromatischer Mono- und Dicarbonsäuren.

14. Verfahren zur Herstellung einer semikristallinen Pulver-Färbezusammensetzung nach Anspruch 1, das umfaßt:
(1) Auflösung in einem inerten organischen Lösungsmittel, ausgewählt aus aliphatischen Chloriden, Alkylcarbonsäureestern mit 3 bis etwa 10 Kohlenstoffatomen und Mischungen davon, eines teilweise kristallinen Polyesterfarbstoffkonzentrats mit einer inhärenten Viskosität von mindestens 0,2, enthaltend:
(i) Disäuregruppen, bestehend aus mindestens 80 mol-% Terephtalsäuregruppen, 2,6-Naphthalindicarbonsäuregruppen, 1,3-Cyclohexandicarbonsäuregruppen, 1,4-Cyclohexandicarbonsäuregruppen oder einer Mischung davon;
(ii) Diolgruppen, bestehend aus mindestens 50 mol-% Gruppen mit der Formel -O-(CH₂)ₚ-O- wobei p 2 bis 12 ist und
(iii) mindestens 5,0 %, bezogen auf das Gewicht der Zusammensetzung, Gruppen einer oder mehrerer difunktionaler Farbstoffverbindungen, die bei Polyester-Polymerisierungsbedingungen stabil sind, vorausgesetzt daß die Farbstoffverbindung nicht eine Methinverbindung ist.
(2) Ausfällung aus der Lösung des Schritts (1) der semikristallinen Farbstoffzusammensetzung in feinverteilter Form, bestehend aus Teilchen mit einer durchschnittlichen Teilchengröße von weniger als 50 Mikrometer.

15. Verfahren nach Anspruch 14, wobei der teilweise kristalline Polyester umfaßt:
(i) Disäuregruppen, bestehend aus mindestens 80 mol-% Terephthalsäuregruppen, 2,6-Naphthalindicarbonsäuregruppen, 1,3-Cyclohexandicarbonsäuregruppen, 1,4-Cyclohexandicarbonsäuregruppen oder eine Mischung davon;
(ii) Diolgruppen, bestehend aus mindestens 50 mol-% Gruppen mit der Formel -O-(CH₂)ₚ-O- wobei p 4 bis 12 ist und
(iii) mindestens 5,0 Gewichtsprozent, bezogen auf das Gewicht der Zusammensetzung, Gruppen eines oder mehrerer difunktionaler Farbstoffverbindungen, die unter Polyester-Polymerisierungsbedingungen stabil und in den Polyester copolymerisiert sind, vorausgesetzt daß die Farbstoffverbindung nicht eine Methinverbindung ist.

16. Verfahren nach Anspruch 14, wobei der teilweise kristalline Polyester eine inhärente Viskosität von mindestens 0,2 und eine Schmelztemperatur von mindestens 110°C hat, und welches umfaßt:
(i) Disäuregruppen, bestehend aus mindestens 80 mol-% Terephthalsäuregruppen;
(ii) Diolgruppen bestehend aus mindestens 80 mol-% Resten der 1,4-Butandiolgruppen und
(iii) etwa 10 bis 40 Gewichtsprozent, bezogen auf das Gewicht der Zusammensetzung, Gruppen eines oder mehrerer difunktionaler Farbstoffverbindungen, die unter Polyester-Polymerisierungsbedingungen stabil und in den Polyester copolymerisiert sind, vorausgesetzt daß die Farbstoffverbindung nicht eine Methinverbindung ist.

17. Verfahren nach Anspruch 14, wobei Schritt (1) die Auflösung einer Schmelze des teilweise kristallinen Polyesters in einem inerten Lösungsmittel, ausgewählt aus C₁-C₄-niederen-Alkylester aromatischer Mono- und Dicarbonsäuren oder Glycolestern von C₁-C₄-aliphatischen-Carbonsäuren, umfaßt.

## Revendications

1. Composition de colorant en poudre semicristalline ayant une dimension moyenne de particules de moins de 50 µm, comprenant un polyester qu'on a modifié par dissolution-cristallisation-précipitation pour lui conférer la cristallinité, contenant à l'état copolymérisé au moins 1,0 % en poids, par rapport au poids de la composition, de résidus d'un ou plusieurs composés colorants difonctionnels qui sont stables dans les conditions de polymérisation du polyester, pourvu que le composé colorant ne soit pas un composé méthinique.

2. Composition de colorant en poudre semicristalline selon la revendication 1, comprenant un polyester normalement amorphe ayant une viscosité inhérente d'au moins 0,2, qu'on a modifié par dissolution-cristallisation-précipitation pour lui conférer la cristallinité, comprenant :
(i) des résidus de diacides comprenant au moins 50 mol % de résidus d'acide téréphtalique et/ou d'acide 2,6-naphtalène-dicarboxylique ;
(ii) des résidus de diols comprenant au moins 50 mol % de résidus d'un diol de formule 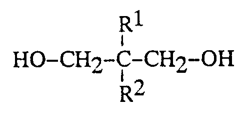 dans laquelle R¹ est un atome d'hydrogène ou un groupe alkyle en C₁-C₄ et R² est un groupe alkyle en C₁-C₄ ; et
(iii) au moins 5,0 % en poids, par rapport au poids de la composition, de résidus d'un ou plusieurs composés colorants difonctionnels qui sont stables dans les conditions de polymérisation du polyester, pourvu que le composant colorant ne soit pas un composé méthinique.

3. Composition de colorant en poudre semicristalline selon la revendication 1, ayant une dimension moyenne de particules de moins de 50 µm, comprenant un polyester normalement amorphe ayant une viscosité inhérente d'environ 0,2 à 0,8 qu'on a modifié par dissolution-cristallisation-précipitation pour lui conférer la cristallinité, comprenant
(i) des résidus de diacides comprenant au moins 80 mol % de résidus d'acide téréphtalique et/ou d'acide 2,6-naphtalène-dicarboxylique;
(ii) des résidus de diols comprenant au moins 80 % mol de résidus d'un diol de formule : 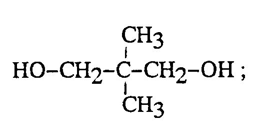 et
(iii) au moins 5,0 % en poids par rapport au poids de la composition de résidus d'un composé colorant difonctionnel qui sont stables dans les conditions de polymérisation du polyester, pourvu que le composé colorant ne soit pas un composé méthinique.

4. Composition de colorant en poudre semicristalline selon la revendication 2, dans laquelle le composé (iii) constitue environ 10 à 40 % en poids de la composition.

5. Composition de colorant en poudre semicristalline selon la revendication 1, ayant une dimension moyenne de particules de moins de 50 µm, comprenant un polyester partiellement cristallin qu'on a modifié par dissolution-cristallisation-précipitation pour lui conférer la cristallinité, comprenant :
(i) des résidus de diacides comprenant au moins 80 mol % de résidus d'acide téréphtalique, de résidus d'acide 2,6-naphtalène-dicarboxylique, de résidus d'acide 1,3-cyclohexane-dicarboxylique, de résidus d'acide 1,4-cyclohexane-dicarboxylique ou d'un de leurs mélanges ;
(ii) des résidus de diols comprenant au moins 50 mol % de résidus de formule -O-(CH₂)ₚ-O- dans laquelle p est un nombre de 2 à 12; et
(iii) au moins 5,0 % en poids par rapport au poids de la composition de résidus d'un ou plusieurs composés colorants difonctionnels qui sont stables dans les conditions de polymérisation du polyester, pourvu que le composé colorant ne soit pas un composé méthinique.

6. Composition de colorant en poudre semicristalline selon la revendication 5, comprenant :
(i) des résidus de diacides comprenant au moins 80 mol % de résidus d'acide téréphtalique, de résidus d'acide 2,6-naphtalène-dicarboxylique, de résidus d'acide 1,3-cyclohexane-dicarboxylique, de résidus d'acide 1,4-cyclohexane-dicarboxylique ou d'un de leurs mélanges ;
(ii) des résidus de diols comprenant au moins 50 mol % de résidus de formule -O-(CH₂)ₚ-O- dans laquelle p est un nombre de 4 à 12 ; et
(iii) au moins 5,0 % en poids par rapport au poids de la composition de résidus d'un ou plusieurs composants colorants difonctionnels qui sont stables dans les conditions de polymérisation des polyesters, pourvu que le composé colorant ne soit pas un composé méthinique.

7. Composition de colorant en poudre microcristalline selon la revendication 5, comprenant un polyester ayant une viscosité inhérente d'au moins 0,20 et une température de fusion d'au moins 110°C, comprenant :
(i) des résidus de diacides comprenant au moins 80 mol % de résidus d'acide téréphtalique ;
(ii) des résidus de diols comprenant au moins 80 mol % de résidus de 1,4-butanediol ; et
(iii) environ 10 à 40 % par rapport au poids de la composition de résidus d'un ou plusieurs composés colorants difonctionnels qui sont stables dans les conditions de polymérisation du polyester pourvu que le composé colorant ne soit pas un composé méthinique.

8. Composition de colorant en poudre microcristalline selon la revendication 6, dans laquelle le composant (iii) constitue environ 10 à 40 % en poids de la composition.

9. Procédé pour la préparation d'une composition de colorant en poudre semicristalline selon la revendication 1 qui comprend les étapes suivantes
(1) on dissout dans un solvant organique inerte un concentré de polyester coloré comprenant un polyester contenant à l'état copolymérisé au moins 1,0 % en poids de résidus d'au moins un composé colorant difonctionnel qui sont stables dans les conditions de polymérisation du polyester, pourvu que le composé colorant ne soit pas un composé méthinique ; et
(2) on précipite de la solution de l'étape (1) la composition de colorant semicristallin sous une forme finement divisée comprenant des particules ayant une dimension moyenne de particules de moins de 50 µm.

10. Procédé selon la revendication 9, dans lequel la dissolution est effectuée à une température d'environ 25°C jusqu'au point d'ébullition du solvant inerte et le solvant inerte est choisi parmi les chlorures aliphatiques, les esters d'acides carboxyliques d'alkyles en C₃ à environ C₁₀ et leurs mélanges.

11. Procédé pour la préparation d'une composition de colorant en poudre semicristalline selon la revendication 1, qui comprend les étapes suivantes :
(1) on dissout dans un solvant organique inerte choisi parmi les chlorures aliphatiques, les esters d'alkyles d'acides carboxyliques ayant 3 à environ 10 atomes de carbone et leurs mélanges un concentré de polyester amorphe coloré ayant une viscosité inhérente d'au moins 0,2, comprenant :
(i) des résidus de diacides comprenant au moins 50 mol % de résidus d'acide téréphtalique et/ou de résidus d'acide 2,6-napthalènedicarboxylique ;
(ii) des résidus de diols comprenant au moins 50 mol % de résidus d'un diol de formule : 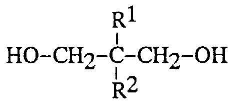 dans laquelle R¹ est un atome d'hydrogène ou un groupe alkyle en C₁-C₄ et R² est un groupe alkyle en C₁-C₄ ; et
(iii) au moins 5,0 % en poids par rapport au poids de la composition de résidus d'un ou plusieurs composés colorants difonctionnels qui sont stables dans les conditions de polymérisation du polyester, copolymérisés dans le polyester, pourvu que le composé colorant ne soit pas un composé méthinique ; et
(2) on précipite de la solution de l'étape (1) la composition de colorant semicristalline sous une forme finement divisée consistant en particules ayant une dimension moyenne de particules de moins de 50 µm.

12. Procédé selon la revendication 11, dans lequel le polyester amorphe a une viscosité inhérente d'environ 0,2 à 0,8 et comprend :
(i) des résidus de diacides comprenant au moins 80 mol % de résidus d'acide téréphtalique et/ou d'acide 2,6-naphtalène-dicarboxylique ;
(ii) des résidus de diols comprenant au moins 80 mol % de résidus d'un diol de formule : 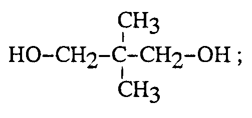 et
(iii) environ 10 à 40 % en poids par rapport au poids de la composition de résidus d'un composé colorant difonctionnel qui sont stables dans les conditions de polymérisation du polyester, copolymérisés dans le polyester, pourvu que le composé colorant ne soit pas un composé méthinique.

13. Procédé selon la revendication 11, dans lequel l'étape (1) comprend la dissolution d'une masse fondue du polyester partiellement cristallin dans un solvant inerte choisi parmi les esters d'alkyles en C₁-C₄, d'acides mono- et dicarboxyliques aromatiques.

14. Procédé pour la préparation d'une composition de colorant en poudre semicristalline selon la revendication 1 qui comprend les étapes suivantes :
(1) on dissout dans un solvant organique inerte choisi parmi les chlorures aliphatiques, les esters d'alkyles d'acides carboxyliques ayant de 3 à environ 10 atomes de carbone et leurs mélanges un concentré d'un polyester partiellement cristallin coloré ayant une viscosité inhérente d'au moins 0,2, comprenant les étapes suivantes :
(i) des résidus de diacides comprenant au moins 80 mol % de résidus d'acide téréphtalique et/ou de résidus d'acide 2,6-napthalènedicarboxylique ;
(ii) des résidus de diols comprenant au moins 50 mol % de résidus d'un diol de formule : -O-(CH₂)ₚ-O- dans laquelle p est un nombre de 2 à 12 ; et
(iii) au moins 5,0 % en poids par rapport au poids de la composition de résidus d'un ou plusieurs composés colorants difonctionnels qui sont stables dans les conditions de polymérisation du polyester, pourvu que le composé colorant ne soit pas un composé méthinique ; et
(2) on précipite de la solution de l'étape (1) la composition de colorant semicristallin sous une forme finement divisée comprenant des particules ayant une dimension moyenne de particules de moins de 50 µm.

15. Procédé selon la revendication 14, dans laquelle le polyester partiellement cristallin comprend :
(i) des résidus de diacides comprenant au moins 80 mol % de résidus d'acide téréphtalique, de résidus d'acide 2,6-naphtalène-dicarboxylique, de résidus d'acide 1,3-cyclohexane-dicarboxylique, de résidus d'acide 1,4-cyclohexane-dicarboxylique ou de leurs mélanges ;
(ii) des résidus de diols comprenant au moins 50 mol % de résidus de formule -O-(CH₂)ₚ-O- dans laquelle p est un nombre de 4 à 12 ; et
(iii) au moins 5,0 % en poids par rapport au poids de la composition de résidus d'un ou plusieurs composés colorants difonctionnels qui sont stables dans les conditions de polymérisation du polyester, copolymérisés dans le polyester, pourvu que le composé colorant ne soit pas un composé méthynique.

16. Procédé selon la revendication 14, dans lequel le polyester partiellement cristallin a une viscosité inhérente d'au moins 0,2 et une température de fusion d'au moins 110°C et comprend :
(i) des résidus de diacides comprenant au moins 80 mol % de résidus d'acide téréphtalique ;
(ii) des résidus de diols comprenant au moins 80 mol % de résidus de 1,4-butanediol ; et
(iii) environ 10 à 40 % en poids par rapport au poids de la composition d'un ou plusieurs composés colorants difonctionnels qui sont stables dans les conditions de polymérisation du polyester copolymérisés dans le polyester, pourvu que le composé colorant ne soit pas un composé méthinique.

17. Procédé selon la revendication 14, dans lequel l'étape (1) comprend la dissolution d'une masse fondue du polyester partiellement cristallin dans un solvant inerte choisi parmi les esters d'alkyles inférieurs en C₁-C₄ d'acides mono-et dicarboxyliques aromatiques ou les esters de glycols d'acides carboxyliques en C₁-C₄.
